# Europäisches Patentamt

# European Patent Office

# Office européen des brevets

(11) Publication number: **0 203 162 B1**

# EUROPEAN PATENT SPECIFICATION

(45) Date of publication of patent specification: **04.03.92**

(51) Int. Cl.⁵: **A61M 5/14**

(21) Application number: **85906142.6**

(22) Date of filing: **29.11.85**

(86) International application number:
**PCT/US85/02357**

(87) International publication number:
**WO 86/03417 (19.06.86 86/13)**

Divisional applications 87201291, 87201290 filed on 07.07.87.

(54) **HOUSING ENABLING PASSIVE MIXING OF A BENEFICIAL AGENT WITH A DILUENT.**

(30) Priority: **03.12.84 US 721991**

(43) Date of publication of application:
**03.12.86 Bulletin 86/49**

(45) Publication of the grant of the patent:
**04.03.92 Bulletin 92/10**

(84) Designated Contracting States:
**BE CH DE FR GB IT LI NL SE**

(56) References cited:
**US-A- 4 465 471          US-A- 4 484 909
US-A- 4 511 352          US-A- 4 511 353
US-A- 4 533 348          US-A- 4 534 757
US-A- 4 552 555**

(73) Proprietor: **BAXTER INTERNATIONAL INC. (a Delaware corporation)
One Baxter Parkway
Deerfield Illinois 60015(US)**

(72) Inventor: **ZDEB, Brian
2 E. Lake Shore
Round Lake Park, IL 60073(US)**
Inventor: **PEARSON, Steve
34520 N. Hickory Court
Ingleside, IL 60041(US)**
Inventor: **SLATER, Glenn, L.
27632 W. Luana
Ingleside, IL 60041(US)**

(74) Representative: **MacGregor, Gordon et al
ERIC POTTER & CLARKSON St. Mary's Court
St. Mary's Gate
Nottingham, NG1 1LE(GB)**

Rank Xerox (UK) Business Services

## Description

Technical Field of the Invention

The present invention is related to the delivery of a beneficial agent to a patient and is more particularly directed to the passive delivery of a drug to the intravenous system of a patient in a safe and effective manner.

Background of the Invention

Many drugs are mixed with a diluent before being delivered intravenously to a patient. The diluent may be, for example, a dextrose solution, a saline solution or even water. Many such drugs are supplied in powder form and packaged in glass vials. Other drugs, such as some used in chemotherapy, are packaged in glass vials in a liquid state.

Powdered drugs may be reconstituted in a well known manner, utilizing a syringe which is used to inject liquid into the vial for mixing, the syringe eventually withdrawing the mixed solution from the vial. When a drug must be diluted before delivery to a patient the drug is often injected into a container of diluent after it is reconstituted, where the container may be connected to an administration set for delivery to a patient. More specifically, the diluent is often packaged in glass bottles, or flexible plastic containers such as are sold under the names MINI-BAG™ AND VIAFLEX® by Travenol Laboratories, Inc. of Deerfield, Illinois. These containers have administration ports for connection to an administration set which delivers the container contents from the container to the patient. The drug is typically added to the container through an injection site on the container.

Drugs may be packaged separately from the diluent for various reasons. One of the most important reasons is that many drugs do not retain their chemical and physical stability when mixed with a diluent and thus cannot be stored for any substantial period of time. Also, drugs are often packaged separately from the diluent because many firms which manufacture drugs are not engaged in the business of providing medical fluids in containers for intravenous delivery, and vice versa.

Therefore, a doctor, nurse, pharmacist or other medical personnel must mix the drug and diluent. This presents a number of problems. The reconstitution procedure is time consuming and requires aseptic technique. The operator must provide the proper diluent and a syringe before beginning. Often the powdered drug is "caked" at the bottom of the vial. Thus, when liquid is injected into the vial from a syringe the surface area of contact between the liquid and the powdered drug may be quite small initially, thus making the mixing procedure even more time consuming. Because of the limited vial volume, the increasing drug concentration in the diluent makes it harder to finish the reconstitution process. The operator may attempt to solve this by repeatedly injecting solution into the vial, mixing and withdrawing the solution but this makes necessary additional injections and movement of the syringe which increase the likelihood of contamination. Also, it is sometimes difficult to get all of the drug and/or liquid out of the vial, thus increasing the time required to perform the reconstitution procedure.

The reconstitution procedure should be performed under preferably sterile conditions. In addition to such a requirement making the operator justifiably more cautious and consuming more time, sterile conditions are often hard to maintain. In some instances, a laminar flow hood may be required under which the reconstitution procedure is performed.

Some drugs, such as some chemotherapy drugs, are toxic. Exposure of the operator to the drugs during reconstitution may be dangerous, especially if the operator works with such drugs on a daily basis and is repeatedly exposed to them.

A further problem is that the reconstitution procedure provides a source of confusion as to which container contains which drug. The diluent container should be marked with the drug with which it has been injected and the name of the patient to whom it should be delivered.

After a drug is reconstituted and withdrawn into a syringe barrel, the drug may in some instances be injected immediately into the intravenous system of a patient. More typically however, the reconstituted drug is injected from the syringe into a larger container of solution as discussed above, for connection to an intravenous administration set. This is because often the drug reconstituted in the syringe is still at a concentration so high as to cause local toxicity in the veins of a patient near the injection site where the needle pierces the skin. This may create severe vein irritation which may be medically harmful. Additionally, while the proper dose of medication is in the syringe, immediate injection into the patient's blood stream may create a condition of systemic toxicity wherein the level of drug concentration in the patient's entire blood stream is dangerously high. Yet another reason for not making the injection from the syringe directly into the patient is that it creates an additional injection site into the patient, which may be painful for the patient and provides another opportunity for infection.

For these reasons, the reconstituted drug is more typically injected into a diluent container.

A patient may typically be administered a dextrose or saline solution from a large volume parenteral container, for example, such as a one liter container, delivered through an administration set such as a CONTINU-FLO® administration set sold by Travenol Laboratories. If the reconstituted drug were injected into the large volume parenteral container, delivery of the drug would usually be delivered over too long a time period. Often, these large volume fluids are delivered at very slow flow rates.

More typically, the reconstituted drug is injected into a small volume parenteral container, such as a fifty milliliter container sold by Travenol Laboratories. This MINIBAG™ container is hung at a higher elevation than the large volume parenteral container and is connected by a secondary administration set to an injection site on the primary administration set. Because it is maintained at a higher elevation, the reconstituted drug in the small volume container is delivered, after which fluid from the large volume container begins to flow once more.

A closed reconstitution delivery system is disclosed in U.S. Patent Nos. 4,410,321; 4,411,662; 4,432,755; and 4,458,733, all assigned to Baxter Travenol Laboratories Inc., the assignee of the present invention. As shown therein, a container includes a drug and a diluent in separate compartments which are reconstituted in a closed system before the drug is delivered to the patient. Typically, the container is connected to an administration set which is connected at its other end to the primary administration set, such as with the small volume parenteral container described above. The container shown in these patents solves many of the problems associated with syringe reconstitution. The product does however necessitate a series of reconstitution steps which must be performed by the nurse or other operator prior to delivering the fluid from the container.

Delivery of a drug or other beneficial agent in a manner not requiring reconstitution steps by an operator is shown in U.S. Patent Nos. 4,424,056; 4,432,756; 4,439,183; 4,474,574; 4,479,793; and 4,479,794 and Canadian Patent No. 1,173,795, assigned to Alza Corporation of Palo Alto, California. As disclosed in those patents, a parenteral delivery system is disclosed which has a formulation chamber therein for administering a beneficial agent such as a drug. The system is advantageous in that it provides for reconstitution of the drug by fluid flowing from a large volume parenteral container for example, through the administration set containing the formulation chamber with the drug therein. The system intends to eliminate the need for the time consuming reconstitution procedure described above and appears to eliminate the problems associated with the reconstitution procedure.

Another passive reconstitution system is disclosed in European Patent Application No. 0059694 to Aktiebolaget Hassle of Sweden.

Still another device for delivering a drug "in-line", i.e., in the administration set, is disclosed in Australian Patent No. 15762/83 and corresponding European Patent Application No. 0100296, assigned to Ciba Geigy AG of Switzerland. The device holds the drug and includes a section through which the liquid passes in a direction substantially opposite to the general direction in which liquid flows to the patient.

Yet another system which attempts to provide for drug reconstitution in-line without manual reconstitution by a nurse or other operator is shown in U.S. Patent No. 4,465,471, assigned to Eli Lilly and Co. of Indianapolis, Indiana. That patent discloses constructions for a receptacle in the administration set itself. A separate cartridge containing the drug to be reconstituted and delivered to the patient is plugged into the receptacle. As liquid enters the cartridge for reconstitution of the drug and subsequent delivery out of the cartridge and receptacle and into the patient, some or most fluid continues to flow through the administration set, bypassing the cartridge entirely.

The pre-characterising part of Claim 1 is based on US-A-4 465 471 and the distinguishing features of the invention are set out in the characterising part of Claim 1.

Some of the documents, such as U.S. Patent Nos. 4,424,056; 4,479,793; and 4,479,794, are also directed to systems having a beneficial agent placed "in-line" in an administration set for mixing of the agent and delivery to a patient, wherein the delivery of the agent may be made in a given volume of fluid. Also, a valve controlling fluid flow may be manually operated to deliver the agent in a manner which can be made dependent upon fluid flow.

It is believed that all of the automatic reconstitution type systems suffer from a critical disadvantage which does not take into account typical conditions in a hospital setting. The critical disadvantage is that at low flow rates, there is a danger that the concentration of drug in the fluid being delivered to the patient will become dangerously high, resulting in local toxicity to the patient near the point of introduction into the body.

Nurses typically work with heavy work loads and need to react quickly to emergency situations. It is possible that a nurse setting up one of the passive type delivery systems mentioned above would need to leave the patient to respond to an emergency elsewhere. The nurse may attempt to keep the "status quo" by turning off fluid flow or turning it very low before rushing away from the patient's bed side. Alternatively, the nurse may forget to set an adequately high flow rate. Yet another possibility is that the flow rate may decrease over time as the fluid is being delivered to the patient because of, for example, changes in the administration set tubing lumen as restricted by a controller such as a roller clamp, over time, or changes to the system caused by movement of the patient or the delivery system or both.

It is believed that the possibility of a situation existing with a low flow rate in a passive type drug reconstitution system is significant. It is further believed that the resulting harm to the patient may be severe.

Existing cartridge type device designs may suffer from drawbacks, such as the need for an air eliminating device within the cartridge to permit the device to operate, thereby raising the cost of the cartridge; the need for a liquid-pervious barrier to the dry medicine in the cartridge; or the existence of flow patterns which do not appear to effectuate the efficient delivery of a large percentage of the drug dosage in the required time period.

Existing cartridge device designs do not direct all fluid flow through the cartridge, which results in a more complicated delivery system that is harder to control with different drugs and may require more than one receptacle configuration depending on the kind of drug in the inserted cartridge.

Existing cartridge device designs do not provide for fluid flow around all the beneficial agent in the cartridge from the beginning of fluid flow therethrough, resulting in inconsistent mixing over time.

Existing cartridge device designs do not include means for preventing insertion of the cartridge into the receptacle in an improper manner and do not include any visual indicator that the drug dose has been mixed and delivered downstream.

## Summary of the aims of the Invention

The present invention solves the medically unacceptable problem of excessively high drug concentrations near the entry point into a patient's intravenous system at low fluid flow rates with a passive reconstitution type drug delivery system. The apparatus of trip present invention recognizes and includes the advantages of a passive drug delivery system which eliminates the time consuming manual reconstitution procedure and the problems associated with manual reconstitution.

The apparatus of the present invention recognizes and includes the advantages of a passive system for delivery of a beneficial agent at a rate which is substantially independent of fluid flow rate.

The apparatus of the present invention effectively eliminates the problem of dangerously high delivery concentration, or local toxicity, which is associated with known passive type drug delivery systems directed to the delivery of a beneficial agent in a manner essentially independent of the flow rate.

The housing of the present invention provides for mixing of a therapeutically acceptable dose of the beneficial agent quickly enough to be within a medically acceptable time period such as about thirty minutes for example.

In one embodiment of the invention the housing includes a standard drug vial as the housing cartridge. A third, separate, intermediate portion is provided for establishing fluid flow between the cartridge and the remainder of the housing.

In one embodiment of the invention, the "in-line" receptacle includes a fluid inlet, a receiving segment, a discharge segment and an outlet, such that fluid need not enter the discharge segment to reach the outlet when the cartridge is not plugged into the receptacle.

The receptacle may include a unique flow director including a resilient tube with a flow-bypass opening therein, such that when the cartridge is not plugged into the receptacle fluid entering the receptacle flows through the resilient flow director, out the flow-bypass opening and through the outlet. When the separate cartridge is plugged into the receptacle, the resilient tubular portion of the flow director seals around the outer periphery of a cartridge cannula inserted therein so that virtually all fluid entering the receptacle flows sequentially through the receiving segment, the flow director tube, the cartridge cannula, the cartridge chamber, the discharge segment and the outlet, the receptacle being virtually devoid of fluid flow through the flow director flow bypass opening. In the preferred embodiment, the flow director intercepts but does not occlude the discharge segment in the housing.

Connecting means are provided between the separate receptacle and cartridge and may include first and second cross-over segments. The first cross-over segment is disposed between and adapted for providing fluid communication between the downstream end of the receiving segment and the upstream end of the chamber. The second cross-over segment is disposed between and adapted for fluid communication between the downstream end of the chamber and the outlet, and more particularly between the downstream end of the chamber and the upstream end of the discharge segment.

Puncturable means such as injection sites may be included in the cross-over segments, such as in the portions of the cross-over segments disposed within the receptacle. Puncturing means are included, such as cannulas in the cartridge portions of the first and second cross-over segments, adapted for puncturing the injection sites. The above-described flow director may include one of the injection sites.

It is preferred that the cartridge chamber upstream portion Include a funnel-like configuration, widening in the downstream direction in order to provide for better mixing between the fluid and the beneficial agent within the chamber.

The invention is also directed to a housing including a visual indicator of complete mixing of the beneficial agent, such as provided by a floating sphere or spheres within the housing.

The cartridge of the housing of the present invention may include key-way means to prevent improper insertion into the receptacle.

The present invention is also directed to a cartridge for introducing a beneficial agent into an intravenous delivery system. In one embodiment, the cartridge includes two cannulas for establishing fluid flow with the fluid conduit by piercing two injection sites in the fluid conduit. When the cartridge is connected to the fluid conduit, virtually all fluid flowing in the conduit flows sequentially through a first cartridge cannula, the cartridge chamber, a second cartridge cannula and then back into the fluid conduit.

The present invention is also directed to a method of mixing a beneficial agent with a fluid.

Description of the Drawings

FIG. 1 is a plan view of an intravenous delivery system including a fluid source and a fluid conduit, including the apparatus of the present invention;

FIG. 2 is a plan view of a prior art drug delivery system, utilizing large and small volume parenteral solution containers maintained at different elevations;

FIG. 3 is a cross-sectional view of a housing, including a receptacle "in-line" in an administration set and a separate cartridge adapted for receiving a beneficial agent;

FIG. 4 is a cross-sectional view of the housing illustrated in FIG. 3, but illustrating the cartridge as secured to the receptacle;

FIG. 5 is a perspective view of another housing;

FIG. 6 is a cross-sectional view of the housing illustrated in FIG. 5;

FIG. 7 is a cross-sectional view of still another housing, including an "in-line" receptacle, a separate intermediate portion and a separate cartridge adapted for receiving a beneficial agent; and

FIG. 8 is a cross-sectional view of the housing illustrated in FIG. 7, but with the intermediate portion of the receptacle secured to both the "in-line" portion of the receptacle and the cartridge.

Detailed Description of Examples Embodying the Best Mode of the Invention

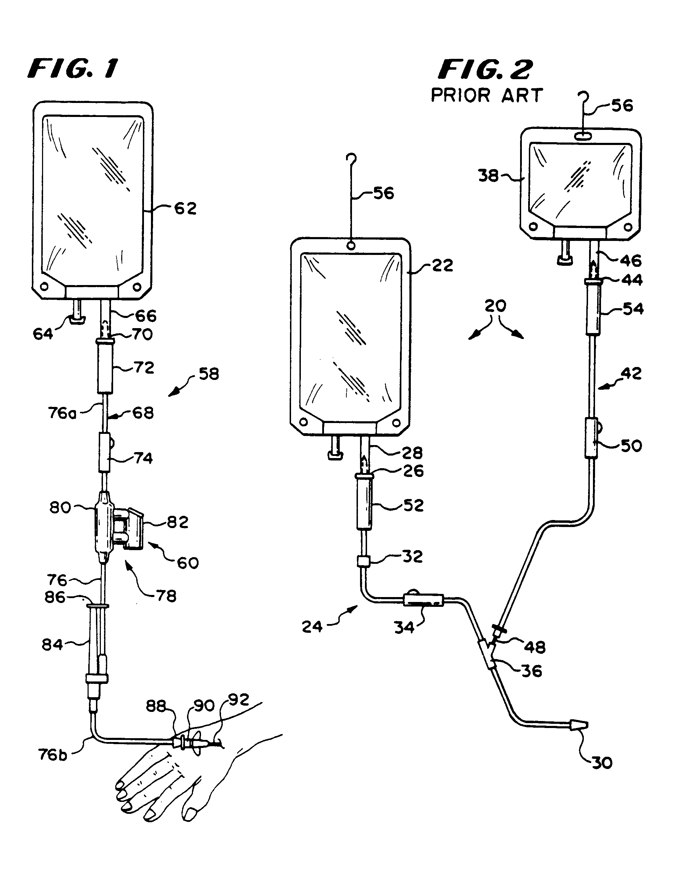

Referring to FIG. 2, there is disclosed a prior art intravenous delivery system 20 including a first fluid source such as a first fluid container 22 having a liquid therein such as water, a dextrose solution or saline solution for example. A first administration set 24 is connected by a first set spike connector 26 at its proximal end to a first container administration port 28 on the first container 22, and by a first set Luer connector 30 to a catheter 31 which enters the patient's intravenous system through the skin.

The first administration set 24 includes a check valve 32, a first flow regulating means such as a first set roller clamp 34 and a "Y" adapter injection site 36. The prior art intravenous delivery system 20 further includes a second fluid source such as a second fluid container 38 which contains a diluent such as sterile water, dextrose, or saline solution for example. A reconstituted drug may be injected into the second fluid container 38 by means of the second container injection site 40.

A second administration set 42 is connected by means of a second set spike connector 44 at its proximal end to the second container administration port 46, and by a needle 48 at its distal end to the first administration set 24 through the "Y" adapter injection site 36. The second administration set 42 includes a second flow regulating means such as a second set roller clamp 50. Each of the first and second administration sets 24, 42 includes a drip chamber 52, 54 respectively by which fluid flow through the sets 24, 42 may be determined by counting drops entering the drip chambers.

The first and second fluid containers 22, 38 may be hung by metal hangers 56 from an IV equipment pole (not shown). The second fluid container 38 is hung at a higher elevation than the first fluid container 22 so that the drug and diluent in the second container 38 will interrupt delivery of fluid in the first container 22 because of a higher head pressure. After the fluid in the second container 38 is delivered, delivery of fluid from the first container 22 to the patient resumes.

In contrast, there is shown in FIG. 1 an intravenous delivery system 58 including an apparatus 60. A fluid source such as a container 62 contains a fluid, such as sterile water, dextrose solution or saline solution for example, to be delivered to the patient. The container 62 includes an injection site 64 and an administration port 66. A fluid conduit including an administration set 68 includes a spike connector 70 at its proximal end for piercing a membrane in the administration port 66 to permit fluid to flow from the container 62 to the set 68. Downstream of the spike connector 70 is a drip chamber 72 by which a nurse or other operator may count drops per unit of time to determine the fluid flow rate through the delivery system 58. A flow regulating device such as a roller clamp 74 is disposed about plastic tubing 76 downstream of the drip chamber 72. The apparatus 60 is disposed "in-line" in the fluid conduit.

The apparatus 60 includes a housing 78. It is preferred that the housing include a receptacle 80 which is disposed in the fluid conduit tubing 76 and a separate, plug-in cartridge 82. Downstream of the housing 78 is a fluid filter 84 which may be, for example, a hollow fiber filter. The fluid filter 84 includes air eliminating means such as an air eliminating membrane 86 for eliminating air entrained in the fluid conduit. Air entrained in the fluid conduit exits the conduit into the environment through the air eliminating membrane 86.

The distal end of the administration set 68 includes a male Luer adapter 69 which connects to a catheter hub 90 of a catheter 92, which enters the patient's intravenous system through the skin.

Many other configurations of the administration set 68 for use with the apparatus 60 are possible. For example, the flow regulating means may be a separate pump into which the tubing 76 is mounted, eliminating the need for the roller clamp 74.

In this disclosure the housings and apparatus are discussed with reference to intravenous delivery systems and the intravenous system of a patient. However, the housings and apparatus may be used for the introduction of a beneficial agent into a patient at locations other than the patient's intravenous system. "Intravenous" is meant to include those other locations. The apparatus and housings are adapted for receiving a beneficial agent. "Beneficial agent" is meant to include diagnostic substances as well as substances intended to have a medically beneficial effect.

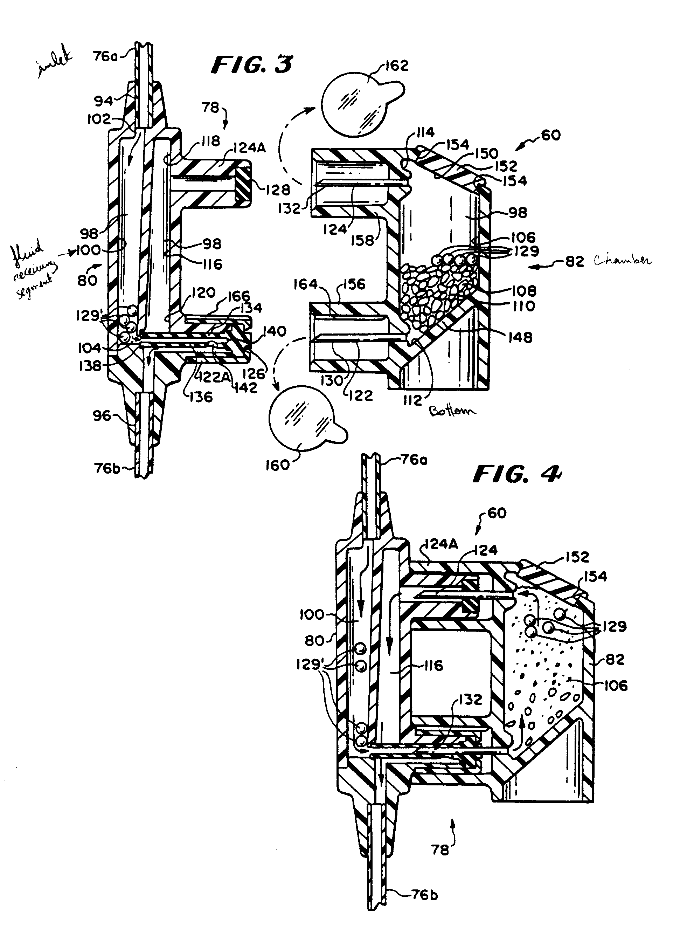

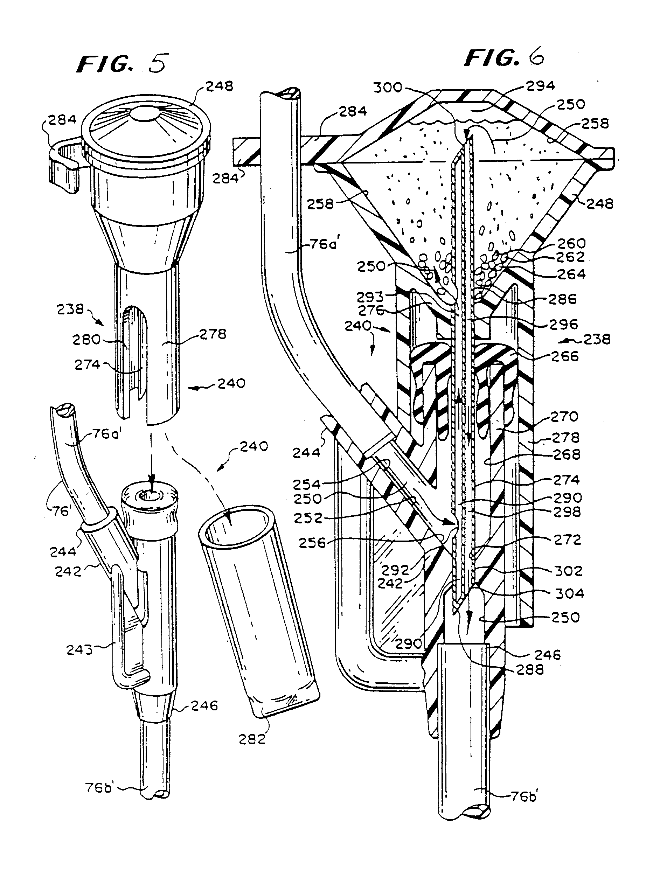

Referring now to FIGS. 3 and 4, there is illustrated the apparatus 60. The apparatus 60 includes a housing 78 which includes a receptacle 80 including an inlet 94 connected to an upstream portion 76a of the fluid conduit and an outlet 96 connected to a downstream portion 76b of the fluid conduit. The housing 78 includes a separate cartridge 82 which is plugged into the receptacle 80 to form the complete housing 78.

The housing 78 defines a fluid pathway 98 therethrough. The inlet 94 and the outlet 96 define part of the fluid pathway 98. The fluid pathway forms part of the fluid conduit of the intravenous delivery system 58.

The housing includes a fluid receiving segment 100 having an upstream end 102 in fluid communication with the inlet 94, and a downstream end 104. A chamber 106 adapted for receiving a beneficial agent 108 and a carrier 110 is defined by the cartridge 82. The chamber 106 defines part of the fluid pathway 98 in the housing 78. As shown in FIGS. 3 and 4, the agent and carrier are in pellet form, but other forms are also possible, as discussed below.

The chamber includes an upstream end 112 adapted for fluid communication with the receiving segment downstream end 104. The chamber also includes a chamber downstream end 114.

The housing 78 further defines a discharge segment 116 of the fluid pathway 98, including a discharge segment upstream end 118 adapted for fluid communication with the chamber downstream end 114, and a discharge segment downstream end 120 in fluid communication with the outlet 96.

The housing 78 further includes connecting means in both the receptacle 80 and the cartridge 82 for securing the cartridge to the receptacle and providing for fluid flow therebetween. The connecting means includes first and second cross-over segments 122, 124. The first cross-over segment 122 is disposed between and adapted for providing fluid communication between the receiving segment downstream end 104 and the chamber upstream end 112.

The second cross-over segment 124 is disposed between and adapted for fluid communication between the chamber downstream end 114 and the discharge segment upstream end 118. The first and second cross-over segments 122, 124 are each disposed in both the receptacle 80 and the cartridge 82.

Puncturable means are disposed in and block both the first and second cross-over segments 122, 124 in the housing 78. The puncturable means used in the housing 78 are resilient, rubber-like injection sites 126, 128 disposed in the first and second cross-over segments 122, 124 respectively. The injection sites are mounted at the ends of the receptacle portions of the first and second cross-over segments and form part of the connecting means between the cartridge and receptacle.

Puncturing means are provided in both the first and second cross-over segments 122, 124 for puncturing the puncturable means. In the housing 78, the puncturing means comprise pointed, hollow cannulas 130, 132 mounted in the cartridge portion of the first and second cross-over segments 122, 124 respectively. The cannulas 130, 132 form part of the connecting means. When the cartridge and receptacle are urged together, the cannulas 130, 132 pierce the injection sites 126, 128 respectively, thereby placing the receiving segment 100, the chamber 106 and the discharge segment 116 in fluid communication.

The first cross-over segment injection site 126 is part of a flow director 134 which is a puncturable means. In addition to the injection site 126, the flow director 134 includes a tube 136 having an upstream end 138 fluid-sealingly connected to the receiving segment downstream end 104. The injection site 126 closes the opposite end 140 of the tube 136. It is preferred that the flow director 134, including the tube 136 and the injection site 126 be made as a single piece.

The flow director 134 defines a flow bypass opening 142 in the wall of the tube 136. It is preferred that the tube 136 is resilient and sized so as to seal about the outer periphery of the cannula 130 which pierces the injection site 126, as illustrated in FIG. 4. The flow director 134 intersects but does not occlude the discharge segment 116.

Referring to FIG. 3, when the cartridge 82 is not plugged into the receptacle 80, fluid flowing from the fluid source through the fluid conduit 76 flows from the conduit upstream portion 76a, through the Inlet 94 and into the receiving segment 100 of the fluid pathway 98. The fluid flows out the receiving segment downstream end 104, into the flow director 134, and then out of the flow director 134 through the flow bypass opening 142, whereupon it continues to flow out the outlet 96 into the downstream portion 76b of the plastic tubing 76.

Referring now to FIG. 4, when the chamber is in fluid communication with the receiving segment and the discharge segment, the tube 136 seals around the outer periphery of the cannula 130 so that fluid entering the flow director 134 does not exit through the flow bypass opening 142. Instead, fluid entering the flow director 134 flows through the cannula 130 into the chamber 106. The fluid continues to flow through the cannula 132, into the discharge segment 116. Fluid in the discharge 116 flows around the flow director 134, into the outlet 96 and the downstream portion 76b of the conduit. It will be seen from FIG. 4 that the direction of fluid flow through at least a portion of the chamber is in a direction generally opposite to the direction of fluid flow from the fluid source to the patient. In the housing 78, fluid flow through the chamber 106 is in a generally upward direction, which is generally opposite to the downward direction of fluid flow from the container 62 to the patient.

As fluid flows through the chamber 106, the beneficial agent 108 in the chamber 106 mixes with the fluid and is subsequently delivered therewith to the patient. As will be explained below, the beneficial agent 108 may be placed in the chamber 106 alone or combined with a dissolvable or nondissolvable carrier material 110. The beneficial agent 108 or the combined beneficial agent and carrier 108, 110 may be in pellet form as illustrated in FIGS. 3 and 4 or in a larger tablet form or in powder form with very small separate particles.

It has been found that the housing 78 permits passive drug delivery. The nurse, pharmacist or other operator need not go through a series of manual reconstitution steps. Once the cartridge 82 is plugged into the receptacle 80 and fluid begins to flow, the drug or other beneficial agent 108 is automatically mixed in with the fluid for delivery to the patient.

The upward flowpath in the chamber 106 of the housing 76 is important. Because the downstream or discharge end 114 of the chamber 106 is at the top of the chamber 106, the fluid at the downstream end 114 of the chamber has a concentration of beneficial agent 108 lower than if the downstream end of the chamber were at a lower elevation than the upstream end of the chamber. This assists in creating a housing 78 which permits delivery of the beneficial agent 108 in a manner substantially independent of the fluid flow rate so that the amount of beneficial agent 108 delivered per unit time period is substantially a constant for a given housing and beneficial agent.

Thus, the housing 78 allows for delivery of the beneficial agent 108 in a time period short enough to be therapeutically effective, such as between twenty and forty minutes. This time period is not intended to be a limitation but does represent a typically desirable delivery period for many intravenously delivered drugs. The housing 78 also prevents the drug from being delivered too quickly at high flow rates so that the medically unacceptable condition of systemic toxicity discussed above will not occur. Stated differently, the housing 78 prevents the patient's blood stream from having a dangerously high concentration of beneficial agent 108.

The upward flowpath in the chamber 106 also aids in mixing by creating swirling of the fluid as it flows upwardly through and around the beneficial agent 108. The upward flow path also assists in forcing downstream out the outlet 96 the air entrained in the housing and in the system upstream of the housing. This permits easy priming of the housing with the cartridge therein. In fact, the cartridge 82 is self-priming. Furthermore, the upward flow path in the chamber 82 of the housing 78 eliminates the need for an air vent or other air eliminating means in the cartridge 82 itself.

It is believed that air eliminating means should be included in the fluid conduit of the intravenous delivery system 58. As shown in FIG. 1, the air eliminating means may simply be the air eliminating membrane 86 of a commercially available fluid filter 84. The air eliminating means eliminates air entrained in the fluid conduit, including the housing. Although it is possible for the air eliminating membrane to be disposed within the housing, it is preferable that it be disposed downstream of the housing. By so doing, the system 58 takes advantage of the air eliminating membrane 86 already in the known fluid filter 84 which would probably be desirable in the system even without the housing. By permitting the air eliminating membrane to be external of the housing 78, and particularly external of the cartridge 82 with the chamber 106 therein, the manufacturing costs of the housing and particularly the cartridge 82 are significantly reduced.

Preferably, the chamber upstream portion adjacent the chamber upstream end 112 has a funnel-like configuration, such as the sloping upstream chamber wall 148. As seen in FIGS. 3 and 4, the funnel-like shape widens in the downstream direction. This configuration aids in the mixing process and helps to direct fluid flow around all of the beneficial agent 108, from the beginning of fluid flow through the chamber. Also preferable is a funnel like configuration adjacent the chamber downstream end 114, such as the sloping, downstream chamber wall 150 that creates a substantially funnel shape which narrows in the downstream direction. Such a structure assists in aiding fluid flow out of the chamber 106.

An additional feature of the housing 78 is a chamber plug 152 sealing a chamber access port 154. The beneficial agent 108 may be placed into the chamber 106 through the chamber access port 154. This design permits manufacture of the housing 78 with the beneficial agent 108 therein, or manufacture of a cartridge 82 which may be delivered to the hospital without a beneficial agent therein. The hospital pharmacist or the operator may then simply take out the plug, insert the beneficial agent 108 and reseal the chamber plug 152 in the access port 154, under aseptic conditions. The chamber plug 152 may be a resilient, puncturable, resealable material, to enable filling the chamber with a syringe and needle through the plug 152 in those cases where the beneficial agent is added to the cartridge 82 in the liquid state.

If the cartridge 82 is manufactured with the beneficial agent therein, the plug 152 may be sealed by means of an ultrasonic weld, solvent bonding or other permanent means. If the cartridge 82 is manufactured for addition of a beneficial agent 108 by hospital personnel, the plug 152 may be designed with a tight friction fit preventing contamination of the chamber through the access port 154 after the chamber has been resealed by the pharmacist.

Preferably, the cartridge 82 includes protective sleeves 156, 158 disposed around and spaced from the cannulas 130, 132 respectively, preventing touch contamination. The sleeves 156, 158 may be covered by removable foil tabs 160, 162 respectively, adhesively secured to the sleeves 156, 158. The tabs 160, 162 are removed before the cartridge 82 is inserted into the receptacle 80.

The protective sleeves 156, 158 also serve the important function of properly aligning the cannulas 130, 132 within the injection sites 126, 128. This is especially important for proper insertion in the flow director 134. The sleeves 156, 158 may have an inner diameter of for example about 0.005 to 0.020 inch greater than the outer diameter of the portions 122A, 124A of the first and second cross-over segments respectively on the receptacle 80. Thus, when the cartridge is plugged into the receptacle the cannula 130 will be centered in the injection site 126 so that the cannula 130 enters the inside of the tube 136 in the flow director.

An additional feature of the cartridge 82 is key way means to prevent the cartridge 82 from being secured to receptacle 80 upside down. The key way means includes a key 164 which, as illustrated in FIG. 3, is mounted on the internal wall of the sleeve 156. The key fits into a slot 166 on the portion 122A of the first cross-over segment 122 on the receptacle 80. The key way means could include many other configurations and may be disposed on the second cross-over segment 124 as well as or instead of the first cross-over segment 122. The key way means prevents the second cannula 132 from piercing the one injection site 126 and prevents the first cannula 130 from piercing the other injection site 128.

Preferably, at least a portion of the cartridge 82 is optically transparent so that the chamber 106 and the chamber contents may be clearly viewed. As will be explained further below, a carrier 110 may be placed in the chamber 106 with the beneficial agent 108. This carrier 110 may be relatively inert and not dissolvable within the fluid flowing through the cartridge 82. For this reason, it may be hard to ascertain by visual inspection whether or not the entire dose of the beneficial agent 108 has been mixed and delivered to the fluid conduit downstream of the chamber. However, visual inspection of complete dosage delivery may be a good safeguard in the intravenous system 58.

Therefore, the housing 78 includes visual inspection means which, in addition to the optically transparent cartridge portion, may also include one or more hollow plastic spheres 129 or other floating visual indicator. In the preferred embodiment, several plastic spheres 129 are carried in the chamber 106, each floating in fluids having different ranges of specific gravity. An identical set of spheres 129' are carried in the receiving segment 100. The receiving segment 100 of the receptacle 80 is also made optically transparent to view the spheres 129'. Because the beneficial agent does not enter the receiving segment 100, the number of spheres 129' floating in the receiving segment 100 will be dependent upon the specific gravity of the fluid from the fluid source.

The spheres 129' serve as a reference to determine when the agent has substantially all been mixed and delivered downstream of the chamber 106, by comparing the spheres 129 in the chamber with the spheres 129' in the receiving segment. When the same number of spheres 129, 129' remain floating in both the chamber 106 and the receiving segment 100, one knows that the specific gravities of the fluid in the chamber and the receiving segment 100 are substantially the same, so that at least almost all of the beneficial agent 108 has been transported downstream of the chamber 106. Until the agent 108, and the carrier 110 if dissolvable, leave the chamber 106 the specific gravity of the fluid in the chamber will be greater than the specific gravity of the fluid in the receiving segment, and hence more spheres 129 will be floating in the chamber than in the receiving segment.

Spheres may be disposed in the discharge segment 116 in addition to, or in place of, the spheres 129 in the chamber 106, in order to determine whether beneficial agent 108 remains in the fluid in the discharge segment or whether the agent 108 has passed downstream of the discharge segment. The discharge segment of the receptacle 78 would be optically transparent, and the spheres in the discharge segment 116 also could be compared with the spheres in the receiving segment 100.

The effect of downward flow in the receiving segment 100 on the location of the spheres 129' therein can be minimized by enlarging the volume of the receiving segment. Alternatively, the operator may, by means of the roller clamp 74 for example, momentarily stop fluid flow to compare the spheres 129, 129'.

Another embodiment of the visual indicator means is to inlcude spheres 129 only in the chamber 106 and not in the receiving and discharge segments 100, 116. In this embodiment, the floating specific gravity range for each sphere is preselected according to the beneficial agent 108, and the carrier if dissolvable, to be carried in the chamber 106. As mixing of the agent 108 nears completion, spheres begin to drop, providing an indication that mixing is nearing completion. After mixing of the agent 108 is complete and has been delivered out the chamber 106, the last sphere 129 drops.

Still another embodiment of the visual indicator means is to include a single sphere 129 in the chamber 106, once again floating in fluids down to a preselected minimum specific gravity, depending on the agent 108, and the carrier 110 if dissolvable. In this embodiment, as fluid flows through the chamber 106 and the last portion of the beneficial agent 108 is mixed and delivered downstream, the specific gravity of the fluid in the chamber becomes less and the sphere 129 begins to fall until coming to rest adjacent the bottom, upstream portion of the chamber 106. The visual indicator is intended to show whether or not substantially the entire dose has been mixed and delivered downstream.

Preferably, the floating spheres 129, 129' are made of a material to which small air bubbles do not easily adhere, so that the floating ability of the spheres 129 are not changed thereby.

The housing 78 described above facilitates passive drug reconstitution and delivers drug out the outlet 96 in the manner independent of the fluid flow rate through the housing 78.

Referring now to FIGS. 5 and 6, there is illustrated an apparatus 238 for placement in an intravenous delivery system such as shown in FIG. 1, replacing the apparatus 60. A fluid conduit such as plastic tubing 76' includes upstream tubing portion 76a' adapted for connection to a fluid source and downstream tubing portion 76b' adapted for fluid communication with a patient's intravenous system.

The apparatus 238 includes a housing 240 having a receptacle 242 including an inlet 244 connected to the upstream portion 76a' of the fluid conduit and an outlet 246 connected to the downstream portion 76b' of the fluid conduit. The receptacle 242 may be an injection site of virtually standard construction, such as the "Y" adapter injection site 36 shown in the prior art system of FIG. 2.

The housing 240 includes a separate cartridge 248 which is plugged into the receptacle 242 to form the complete housing 240. The housing 240 may also be constructed so that the cartridge 248 and the receptacle 242 are a unit.

Like the housing 78, the housing 240 defines a fluid pathway 250 therethrough. The inlet 244 and the outlet 246 define part of the fluid pathway 250. The fluid pathway forms part of the fluid conduit of the intravenous delivery system.

The housing includes a fluid receiving segment 252 having an upstream end 254 in fluid communication with the inlet 244, and a downstream end 256. A chamber 258 adapted for receiving a beneficial agent 260 and a carrier 262, such as pellets 264 of sodium ampicillin and porous polypropylene for example, is defined by the cartridge 248. The chamber 258 defines part of the fluid pathway 250 in the housing 240.

The receptacle 242 preferably includes a handle 243 for grasping the receptacle 242 when attaching the cartridge 248. The receptacle 242 includes a polyisoprene or other puncturable, resealable situs 266 closing off an access volume 268 defined by the wall 270 of the receptacle 242. The wall 270 also defines a narrower channel 272 adjacent the outlet 246.

The cartridge 248 includes a double bore needle 274 adapted for piercing the receptacle situs 266. The double bore needle 274 is mounted to the cartridge wall 276 so that both bores of the needle 274 are in communication with the chamber 258. A cartridge sleeve 278 mounted about and spaced from the double bore needle 274 prevents touch contamination of the needle 274 and assures a secure fit of the cartridge 248 to the receptacle 242. A slot 280 in the sleeve 278 fits around the fluid receiving segment 252 of the receptacle 242. A tip protector 282 closes the sleeve 278 and double bore needle 274 during storage and is removed before attaching the cartridge to the receptacle.

The cartridge 248 further includes a holding loop 284 for securing the cartridge to the upstream tubing portion 76a'.

The chamber 258 includes a chamber upstream end 286 adapted for fluid communication with the receiving segment downstream end 256, and a chamber downstream end 294, opposite the upstream end 286.

The first and second bores 290, 298 of the double bore needle 274, along with the situs 266, form connecting means for securing the cartridge 248 to the receptacle 242 and providing for fluid flow therebetween. The connecting means includes first and second cross-over segments which correspond to the first and second bores 290, 298. The first cross-over segment, in this case the first bore 290, is disposed between and adapted for providing fluid communication between the receiving segment downstream end 256 and the chamber upstream end 286.

In the housing 240 the second cross-over segment, in this case the second bore 298, comprises the discharge segment 296, so that the second cross-over segment is disposed between and adapted for fluid communication between the chamber downstream end 294 and the outlet 246. The discharge segment 296 includes a discharge segment upstream end 300 which is the open end of the second bore 298 within the chamber 258, adapted for fluid communication with the chamber downstream end 294. The discharge segment downstream end 302 is defined by the open distal end 304 of the second bore 298.

The access volume 268, as closed by the situs 266, also forms part of both the first and second cross-over segments. Thus, the first and second cross-over segments are each disposed in both the receptacle 242 and the cartridge 248.

The situs 266 comprises puncturable means. The closed distal end 288 of the first bore 290 and the open distal end 304 of the second bore 298, all being part of the double bore needle 274, comprise the puncturing means.

When the cartridge 248 is not plugged into the receptacle 242, fluid flowing from the fluid source through the fluid conduit 76' flows through the housing inlet 244 and into the receiving segment 252. The fluid flows out the receiving segment downstream end 256, whereupon it continues to flow out the outlet 246 into the downstream portion 76b' of the tubing 76'.

When the cartridge 248 is plugged into the receptacle 242 such that the chamber 258 is in fluid communication with the receiving segment 252 and the discharge segment 296, the double bore needle 274 seals against the narrower channel 272 within the receptacle 242 so that fluid entering the receiving segment 252 does not exit immediately into the outlet 246 but must instead first enter the double bore needle 274. Fluid flows from the receiving segment downstream end 256 into the first side opening 292 of the first bore 290 and up the first bore 290. Fluid exits the first bore 290 at the second side opening 293 of the first bore 290, into the chamber 258 at the chamber upstream end 286. The fluid flows up through and around the beneficial agent 260 and carrier 262 upwardly to adjacent the chamber upstream end 294, whereupon it begins to flow downwardly through the discharge segment 296 and then through the outlet 246.

The beneficial agent 260 may be combined with a carrier 262 and may be in powder, pellet, tablet or other form. The carrier may be dissolvable or non-dissolvable. When the limits of agent dissolution provided by the tablet, pellet, powder or other agent or agent/carrier configuration are combined with the limitations of the flowpath in the housing 240, the apparatus 238 thereby includes control means necessary to push the fluid flow rate transition region up into a flow rate range permitting flow rate dependent agent delivery at flow rates high enough to prevent local toxicity.

Even without the control means the housing 240 permits passive drug reconstitution and includes all the advantages provided by an upward flowpath as discussed with reference to the housing 78. Similar to the housing 78, the housing 240 allows for delivery of the beneficial agent 260 in a time period short enough to be therapeutically effective. However, the housing 240 prevents the drug from being delivered too quickly at high flow rates so that the medically unacceptable condition of systemic toxicity will not occur.

As with the housing 78, it is believed that air eliminating means should be included in the fluid conduit of the intravenous delivery system including the housing 240. However, as with the housing 78, the air eliminating means need not be disposed within the housing 240 itself, but rather may be disposed downstream of the housing.

As with the housing 78, the housing 240 may be designed for shipment to a hospital with the beneficial agent therein, or designed for agent filling at the hospital.

The slot 280 in the cartridge sleeve 278 along with the sleeve 278 itself provides a key way to assure that the first side opening 292 is disposed facing the upstream tubing portion 76', although it is believed that even if the first side opening 292 were rotated 180° the housing 240 would still function properly.

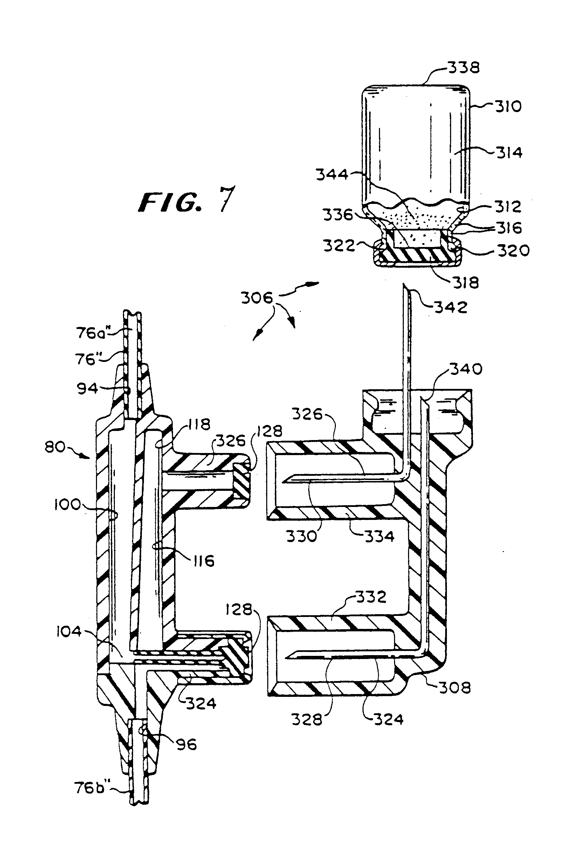

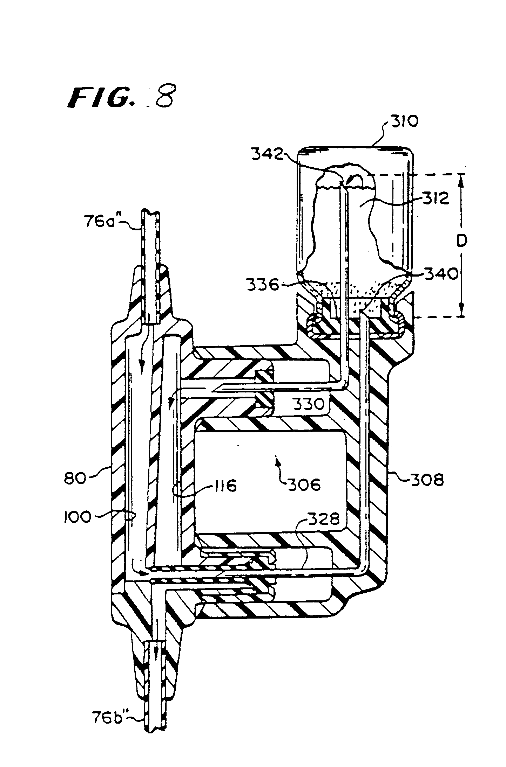

Referring now to FIGS. 7 and 8 there is disclosed yet another housing 306, including a receptacle 80 which may be identical to the receptacle in the housing 78. The receptacle 80 is mounted "in-line" in an intravenous delivery system between upstream and downstream portions 76a'', 76b'' respectively of plastic tubing 76''.

The housing 306 includes a separate receptacle 80, intermediate portion 308 and cartridge 310. The cartridge 310 may be a drug vial of standard construction. The ability to use a drug vial of standard construction represents one of the advantages of the housing 306. FIG. 18 illustrates the three separate units. Before the three units are connected, fluid flows through the receiving segment 100 from the inlet 94 to the outlet 96. FIG. 8 illustrates the housing 306 with the three units 80, 308, 310 connected so that virtually all fluid which flows out of the outlet 96 first flows through all three of the receiving segment 100, the chamber 312 defined by the cartridge 310, and the discharge segment 116, forming a fluid pathway in the housing 306.

When the cartridge 310 is a drug vial of standard construction, it typically includes an optically transparent glass wall 314 defining the chamber 312 and a container neck 316. A stopper 318 or situs of rubber or other puncturable, resealable material is mounted within the mouth 320 defined by the neck 316. A metal band 322 is secured about the mouth 320, retaining the stopper 318 in the cartridge 310.

Connecting means allow for fluid communication between the receptacle 80, the intermediate portion 308 and the cartridge 310. The connecting means includes first and second cross-over segment 324, 326. Portions of both the first and second cross-over segments are included in the receptacle 80. Injection sites 126, 128 block the first and second cross-over segments 324, 326 in the receptacle 80 and serve as the puncturable means as described above with reference to the housing 78. Puncturing means for the injection sites 126, 128 include first and second intermediate cannulas 328, 330 mounted within the intermediate portion 308 and serving as part of the first and second cross-over segments 324, 326 respectively. The intermediate portion 308 includes sleeves 332, 334 mounted about and spaced from the portions of the cannulas 328, 330 respectively which pierce the injection sites 126, 128, preventing touch contamination.

When disposed as shown in FIG. 8, the chamber upstream end 336 is adjacent the rubber stopper 318 and the chamber downstream end 338 is the end opposite the stopper. The first and second cross-over segments 324, 326 include sharpened vial ends 340, 342 for puncturing the stopper 318. The first cross-over segment vial end 340 is sized so as to just pierce the stopper 318, projecting just slightly into the chamber 312. The second cross-over segment vial end 342 projects much further into the chamber 312, closer to the chamber downstream end 338.

The cartridge 310 includes a beneficial agent 344 which is most commonly in a powder form and, as shown in FIG. 8, is disposed within the cartridge neck 316 which has a natural funnel-like configuration.

By placing the first cross-over segment vial end 340 barely within the chamber, a turbulant flow pattern is created around the beneficial agent 344. By placing the second cross-over segment vial end 342 well within the chamber 312, vertically spaced from the first segment vial end 340, an upward flow pattern is created within the chamber 312.

The first cross-over segment 324 provides fluid flow communication between the receiving segment downstream end 104 and the chamber upstream end 336. The second cross-over segment 326 provides for fluid communication between the chamber downstream end 338 and the discharge segment upstream end 118.

The distance between the second segment vial end 342 and the chamber upstream end 336, noted as distance "D", along with the volume of the chamber 312 are two factors which determine the concentration of agent in fluid flowing into the second cross-over segment vial end 342, downstream to the outlet 96. It should be noted that the critical distance "D" also corresponds to the distance between the second side opening 293 and the discharge segment upstream end 300 in the housing 240 of FIGS. 5 and 6.

Raising the first segment vial end 340 will lessen turbulence through the beneficial agent 344 stored in the chamber, lessening the mixing action and thereby relying more on the inherent dissolution rate of the beneficial agent 344 for mixing the agent 344 with the fluid. In addition, the solubility of the beneficial agent and the dose of agent 344 within the chamber may also determine the appropriate distance between the second segment vial end 342 and the stopper 318 (distance "D"), in order to deliver the agent dose within a therapeutically acceptable time period. Listed below are agent, dosage and approximate needle distance "D" and vial (cartridge) volume for each of four drugs tested with a similar housing 306, for delivery of the agent within the prescribed time periods.

| Drug | Dose | Distance "D" | Vial Volume |
|---|---|---|---|
| Ampicillin | 1 gm | 2" (5.08 cm) | 10cc |
| Ampicillin | 2 gm | 2" (5.08 cm) | 20cc |
| Cephalothin | 1 gm | 1" (2.54 cm) | 10cc |
| Cephalothin | 2 gm | 1 1/2" (3.81 cm) | 20cc |
| Cefazolin | 0.5 gm | 1 1/2" (3.81 cm) | 10cc |
| Cefazolin | 1 gm | 1 1/2" " | 10cc |
| Cefazolin | 2 gm | 1 1/2" " | 20cc |
| Ticarcillin | 1 gm | 1" (2.54 cm) | 20cc |
| Ticarcillin | 2 gm | 1 1/2" (3.81 cm) | 20cc |
| Ticarcillin | 3 gm | 2" (5.08 cm) | 30cc |

The above data are intended to be examples only and may vary. Different needle lengths in the same vial volume for the same dosage weights of different drugs are necessitated because of different drug solubilities and rates of dissolution.

The housing 306 may also be constructed in a manner such as would be represented by turning the intermediate portion 308 and the cartridge 310 upside down with respect to the receptacle 80, so that the second cannula 330 is upstream of the first cannula 328.

Furthermore, while the housing 306 is intended for use with a standard drug vial as the cartridge 310, which vials typically have powdered drug therein, the cartridge 310 can instead store a tablet, pellets, powder or other configuration of beneficial agent and carrier as discussed above.

Furthermore, it may be possible, by controlling the variables of chamber volume, cross-sectional configuration of the flow path and locations of the cross-over segment vial ends 340, 342 to create an apparatus which includes the necessary control means to create both first and second delivery modes where the second delivery mode of flow rate dependent agent delivery is at flow rates medically acceptably high enough to prevent local toxicity of agent.

As with the housing 78, the intravenous delivery system including the housing 306 should also include air eliminating means, preferably downstream of the housing 306.

The housing 306 permits passive agent reconstitution and permits the use of standard drug vials used by many companies to contain drugs.

While several embodiments and features have been described in detail herein and shown in the accompanying drawings, it will be evident that various further modifications are possible without departing from the scope of the invention.

**Claims**

1. A housing (78,240,306) adapted for insertion in an intravenous delivery system including a fluid source (62) and a fluid conduit (76,76$^1$,76$^{11}$) for delivering liquid from the fluid source through the fluid conduit to the intravenous system of a patient, the housing being adapted for receiving a beneficial agent (108,236,344) to be mixed with fluid flowing through the fluid conduit, said housing comprising:

   a) a receptacle (80,80$^1$,242) adapted for insertion into the fluid conduit (76,76$^1$,76$^{11}$) and including an inlet (94,244) adapted for connection to an upstream portion of the fluid conduit, an outlet (96,96$^1$,246) adapted for connection to a downstream portion of the fluid conduit, and a fluid-receiving segment (100,252) having an upstream end (102,254), in fluid communication with the inlet (94,244), and a downstream end (104,256),

   b) a separate cartridge (82,82$^1$,248,310) connectible to said receptacle and including a chamber (106,258,312) adapted for receiving the beneficial agent, said chamber including an upstream end (112,286,336) in fluid communication with said inlet (94,244) when connected to the receptacle, the chamber further including a downstream end (114,294,338);

   c) a discharge segment (116,296) including an upstream end (118,300) in communication with said chamber downstream end (114,294), the discharge segment having a downstream end (120,302) in communication with said outlet (96),

   d) such that as fluid flows through said housing, the beneficial agent in said chamber mixes with the liquid and is delivered therewith to the patient,

   e) connecting means (122,122A&124,124A;278&296;324&326) in both said receptacle and said cartridge for securing said cartridge to said receptacle;

   f) whereby when said cartridge is not secured to said receptacle, fluid from the fluid source may still be delivered to the patient through the fluid conduit (76), including said receptacle, with no liquid leaving said receptacle between said receptacle inlet and said receptacle outlet;

   characterised in that:

   g) the upstream end (112,286) of the chamber is in communication with the downstream end (104,256) of the fluid-receiving segment to provide said communication with the inlet (94,244);

   h) whereby, when said chamber is in fluid communication with said receiving segment and said discharge segment, virtually all fluid which flows out of said outlet flows through all three of said receiving segment, said chamber and said discharge segment, forming a fluid pathway in said housing; and

   i) further whereby, with the housing inserted in an intravenous delivery system, said chamber downstream end (114,294) is disposed at an elevation higher than said chamber upstream end (112,286), so that the direction of fluid flow through at least a portion of said chamber is in a generally upward direction, generally opposite to the direction of fluid flow from the fluid source to the patient.

2. The housing in accordance with Claim 1 wherein said receptacle (80) also comprises said discharge segment (116).

3. The housing (306) in accordance with Claim 1, wherein said cartridge (310) includes a wall (314) defining said chamber (312) and a mouth (320) to said chamber, and further comprising a pierceable resealable stopper (318) disposed in and sealing said mouth, said cartridge being adapted to plug into the remainder of said housing so that said chamber is in fluid communication with both said inlet (94) and said discharge segment (116) through said single stopper.

4. The housing in accordance with Claim 3, further comprising a separate, intermediate portion (308) adapted for connection between and fluid communication between said cartridge (310) and said receptacle (80).

5. The housing in accordance with Claim 3 or 4, wherein said cartridge further comprises a metal band (322) about the outer periphery of a neck (316) of said wall (314) defining said mouth, the band locking said stopper (318) within said cartridge.

6. The housing in accordance with Claim 3, 4 or 5 wherein said cartridge is a drug vial (310).

7. The housing in accordance with any preceding claim further comprising;

a) A first connecting segment (122&122A,290,324) disposed between and adapted for providing fluid communication between said inlet (94) and said chamber upstream end (112,286,336); and

b) A second connecting segment (124&124A,298,326) disposed between and adapted for fluid communication between said chamber downstream end (114,286,338) and said outlet (96).

8. The housing as in Claim 7 wherein said second connecting segment (298) comprises said discharge segment (296).

9. The housing in accordance with Claim 7 or 8, wherein said second connecting segment (124,124A) is disposed between and adapted for fluid communication between said chamber downstream end (114) and said discharge segment upstream end (118).

10. The housing in accordance with Claim 7 wherein said first connecting segment (324) includes a segment end (340) and wherein said second connecting segment (328) includes a segment end (342), said ends (340,342) being disposed within said chamber (312).

11. The housing in accordance with Claim 10, wherein said segment end (342) of the second connecting segment (326) and said segment end (340) of the first connecting segment (324) are disposed at different elevations within said chamber (312), said segment end (342) of the second connecting segment (326) being downstream of said segment end (340) of the first connecting segment (324) and being closer to said chamber downstream end (338) than said segment end (340) of the first connecting segment (324).

12. The housing in accordance with Claim 7, 8, 9, 10 or 11, further comprising:

a) Puncturable means (126,128) disposed in and blocking both said first and second connecting segments (122&122A,124&124A); and

b) Puncturing means (130,132) in both said first and second connecting segments for puncturing said puncturable means, such that when said cartridge and said receptacle are urged together, said puncturing means pierces said puncturable means, placing said inlet (94) and said discharge segment (116) in fluid communication.

13. The housing in accordance with Claim 12, wherein said first connecting segment (122,122A) comprises a flow director (134) disposed in said receptacle (80) and including:-

a) A tube (136) having an upstream end (138) fluid-sealingly connected to said receiving segment downstream end (104), and an opposite end (140);

b) A puncturable, resealable injection site (126) defining the puncturable means which blocks said first segment (122&122A) and connected to said opposite end (140) of said tube; and

c) A flow-bypass opening (142) defined in said tube, such that when said cartridge is not secured to said receptacle, fluid flowing into said receptacle flows into said flow director, and out said flow director at said flow-bypass opening, to said outlet.

14. The housing in accordance with Claim 12 or 13, wherein said puncturable means comprise resilient, rubber-like injection sites (126,128).

15. The housing in accordance with Claim 14, wherein said puncturable means comprise pointed cannulas (130,132), said cannulas forming part of said first and second connecting segments (122,124).

16. The housing in accordance with Claim 13, wherein said puncturable means (130) in the first connecting segment (122) comprises a pointed cannula (130), such that when said cartridge is secured to said receptacle, said cannula (130) pierces said flow director injection site (126) and fluid flowing into said receptacle flows into said flow director tube (134), virtually all of said fluid in said flow director tube flowing into said cannula (130), downstream to said outlet (96).

17. The housing in accordance with Claim 16, in which said flow director tube (136) is resilient and sized so as to seal about the outer periphery of said hollow cannula (130), preventing fluid flowing into said flow director tube (136) from flowing out said flow - bypass opening (142) when said cartridge is secured to said receptacle.

**18.** The housing in accordance with Claim 17, wherein said flow director (134) intercepts but dies not occlude said discharge segment (116).

**19.** The housing as in Claim 1, wherein said connecting means includes said discharge segment (296).

**20.** The housing as in Claim 1 wherein said receptacle further comprises a channel (272) cooperating with said discharge segment (296) so that fluid entering the receiving segment (252) does not exit immediately into the outlet (246) but enters the first connecting segment (290).

**21.** The housing as in any preceding claim, wherein said beneficial agent (108) is in powder form.

**22.** The housing in accordance with Claim 1, wherein said chamber upstream portion (148) includes a funnel-like configuration, widening in the downstream direction.

**23.** The housing in accordance with Claim 1, wherein said chamber downstream portion (150) includes a funnel-like configuration, narrowing in the downstream direction.

**24.** The housing in accordance with Claim 1, further comprising air eliminating means (86) in the fluid conduit for eliminating air entrained in the fluid conduit, said air eliminating means being disposed in a position which is one of (a) in said chamber and (b) downstream of said chamber.

**25.** The housing in accordance with any preceding Claim wherein said cartridge (82), when secured to said receptacle (80), is devoid of any means for directing air entrained in the fluid conduit out of said cartridge into the environment external of the intravenous delivery system, without the entrained air first flowing downstream of said cartridge.

**26.** The housing in accordance with any one of Claims 1 to 25 further comprising a portion of the chamber wall being optically transparent, said chamber including a floating Visual indicator (129), therein, other than the beneficial agent, that, because of changing specific gravity of the fluid, changes elevation upon completion of mixing of the beneficial agent and delivery of the agent downstream of said chamber.

**27.** The housing in accordance with any one of Claims 1 to 25, further comprising a portion of the chamber wall being optically transparent, and including a plurality of floating visual indicators (129), other than the beneficial agent, in said chamber, said indicators being able to float in fluids of different specific gravity ranges, such that as mixing nears completion, certain of said visual indicators begin to drop and such that after mixing is complete and after the beneficial agent has been delivered out said chamber, all of said visual indicators have dropped.

**28.** The housing in accordance with any one of Claims 1 to 25 further comprising portions of the housing defining said receiving segment and said chamber being optically transparent, each including a plurality of floating visual indicators (129) therein, other than the beneficial agent, said floating visual indicators within said receiving segment being able to float in fluids of different specific gravity ranges, and said visual indicators within said chamber being able to float in fluids of different specific gravity ranges, but the same ranges as said indicators in said receiving segment, wherein visual confirmation of complete mixing and delivery of the agent downstream of said chamber is made when the same number of visual indicators remain floating in said chamber as in said receiving segment.

**29.** The housing in accordance with any one of Claims 1 to 25, further comprising a portion of the housing defining said discharge segment being optically transparent, said discharge segment including a floating visual indicator (129) therein, other than the beneficial agent, that, because of changing specific gravity in the fluid, changes elevation upon completion of mixing of the beneficial agent and delivery of the agent downstream of said discharge segment.

**30.** The housing in accordance with any one of Claims 1 to 25, further comprising a portion of the discharge segment wall being optically transparent, and including a plurality of floating visual indicators (129), other than the beneficial agent, in said discharge segment, said indicators being able to float within fluids of different specific gravity ranges, such that as mixing nears completion certain of said visual indicators begin to drop and such that after mixing is complete and after the beneficial agent has been delivered out said discharge segment, all of said visual indicators have dropped.

31. The housing in accordance with any one of Claims 1 to 25, further comprising portions of the housing defining said receiving segment said discharge segment being optically transparent, each of said discharge segment and said receiving segment including a plurality of floating visual indicators (129) therein, other than the beneficial agent, said floating visual indicators within said receiving segment being able to float in fluids of different specific gravity ranges, and said visual indicators within said discharge segment being able to float in fluids of different specific gravity ranges, but the same ranges as said indicators in said receiving segment, wherein visual confirmation of complete mixing and delivery of the agent downstream of said discharge segment is made when the same number of visual indicators remain floating in said discharge segment as in said receiving segment.

32. A receptacle (80)adapted for use in a housing according to Claim 1, for insertion in an intravenous delivery system including a fluid source (62) and a fluid conduit (76), for delivering fluid from the fluid source through the fluid conduit to the intravenous system of a patient, said receptacle comprising:

a). An inlet (94) adapted for connection to an upstream portion of the fluid conduit;
b). A fluid receiving segment (100) having an upstream end (102) in fluid communication with said inlet, and a downstream end (104);
c). An outlet (96) adapted for connection to a downstream portion of the fluid conduit;
d). A discharge segment (116) including an upstream end (118,300) in fluid communication with an injection site adapted for communicating with the downstream end (114) of a separate cartridge, and a discharge segment downstream end (120) in communication with said outlet; and
e). A flow director (134), said flow director including:
   i). A tube (136) having an upstream end (138) fluid-sealingly connected to said receiving segment downstream end (104), and an opposite end (140),
   ii). A puncturable, resealable injection site (126) secured to said opposite end (140) for communicating with the upstream end (118) of the separate cartridge, and
   iii). A flow-bypass opening (142) defined in said tube;
f). Such that when the separate cartridge is not connected to said receptacle, fluid entering said receptacle at said inlet flows sequentially through said receiving segment, said flow director tube, said flow director bypass opening and said outlet, wherein said flow director intercepts but does not occlude said discharge segment,
g). Wherein said flow director tube is resilient and effectively seal around the outer periphery of a cartridge cannula inserted through said flow director injection site, such that when the separate cartridge is secured to said receptacle, fluid entering said receptacle at said inlet flows sequentially through said receiving segment, said flow director tube, the cartridge cannula, the cartridge, said discharge segment and said outlet, said receptacle being thereby virtually devoid of fluid flow through said flow director flow-bypass opening;

33. A method for mixing a beneficial agent (108) with fluid flowing from a fluid source (62) through a fluid conduit (76), the steps comprising;
a). Providing a receptacle (80) having a fluid inlet (94) and outlet (96) in the fluid conduit, adapted for having a separate cartridge (82) securable thereto and permitting, when the cartridge is not secured to the receptacle, fluid from the fluid source to be delivered to the patient through the fluid conduit including the receptacle, with no fluid exiting the receptacle between the inlet and outlet;
b). Providing a separate cartridge (82) defining a chamber (106) carrying a beneficial agent (108) therein, the chamber including an upstream end (112) adapted for fluid communication with the receptacle inlet (94) when the cartridge is secured to the receptacle, the chamber further including a downstream end (114);
c). Providing a discharge segment (116) disposed in both the cartridge and the receptacle when the cartridge is operatively secured to the receptacle, the discharge segment further including an Upstream end (118) adapted for communication with the chamber downstream end (114) and a discharge segment downstream end (120) in communication with the outlet (96) when the cartridge is operatively secured to the receptacle; and
d). Operatively securing the cartridge to the receptacle, thereby:
   i). directing virtually all liquid through the receptacle inlet the chamber and the discharge segment before flowing out of the receptacle outlet; and

ii). disposing the chamber downstream end (114) at an elevation higher than the chamber upstream end (112), thereby;

iii). directing fluid flow through at least a portion of the chamber in a generally upward direction, generally opposite to the direction of fluid flow from the fluid source;

iv). such that as fluid flows through the cartridge, the beneficial agent in the chamber mixes with the fluid and is delivered therewith.

34. The method of Claim 33, further comprising removing the cartridge from the receptacle to subsequently permit fluid flow through the receptacle.

35. The method of Claims 34, further comprising connecting another cartridge to the receptacle.

36. The method as in Claim 34, further comprising the step of providing the beneficial agent in the cartridge in powder form.

**Revendications**

1. Boîtier ou enceinte (78,240,306) prévu pour l'insertion dons un système de distribution intraveineuse comprenant une source de fluide (62) et un conduit de fluide (76,76',76") pour distribuer un liquide à partir de le source de fluide, par l'intermédiaire du conduit de fluide, au système intraveineux d'un patient, le boîtier étant prévu pour recevoir un agent de traitement (108,236,344) à mélanger avec le fluide s'écoulant dans le conduit de fluide, ledit boîtier comprenant:

   a) un réceptacle (80,80',242) prévu pour insertion dans le conduit de fluide (76,76',76") et comportant une entrée (94,244) prévue pour le raccordement à une partie amont du conduit de fluide, une sortie (96,96' 246) prévue pour le raccordement à une partie aval du conduit de fluide, et un segment de réception de fluide (100,252) ayant une extrémité amont (102,254), en communication de fluide avec l'entrée (94,244), et une extrémité aval (104,256) ;

   b) une cartouche séparée (82,82',248,310) pouvant être accouplée audit réceptacle et comportant une chambre (106,258,312) prévue pour recevoir l'agent de traitement, ladite chambre comportant une extrémité amont (112,286,336) en communication defluide avec la dite entrée (94,244) lorsqu'elle est raccordée au réceptacle, la chambre comportant en outre une extrémité aval (114,294,338) ;

   c) un segment de sortie (116,296) comportant une extrémité amont (118,300) en communication avec la dite extrémité aval (114,294) de la chambre, le segment de sortie comportent une extrémité aval (120,302) en communication avec ladite sortie (96) ;

   d) de sorte que, lorsque le fluide circule à travers ledit boîtier, l'agent de traitement contenu dans ladite chambre se mélange ou liquide et est distribué avec celui-ci au patient ;

   e) des moyens de connexion (122, 122a et 124, 124a ; 278 et 296 ; 324 et 326) à la fois dans ledit réceptacle et ladite cartouche, pour fixer ladite cartouche audit réceptacle ;

   f) de sorte que, lorsque ladite cartouche n'est pas fixée audit réceptacle, le fluide venant de la source de fluide peut encore être amené au patient par le conduit de fluide (76), comprenant ledit réceptacle, aucun liquide ne quittant ledit réceptacle entre ladite entrée de réceptacle et ladite sortie de réceptacle ; caractérisé en ce que :

   g) l'extrémité amont (112,286) de la chambre est en communication avec l'extrémité aval (104,256) du segment de réception, de fluide pour assurer ladite communication avec l'entrée (94,244) ;

   h) de sorte que, lorsque ladite chambre est en communication de fluide avec ledit segment de réception et ledit segment de sortie, pratiquement tout le fluide qui sort de ladite sortie passe dans la totalité dudit segment de réception, de ladite chambre et dudit segment de sortie, constituant un passage de fluide dans ledit boîtier ; et

   i) de sorte que, en outre, lorsque le boîtier est inséré dans un système de distribution intraveineuse, ladite extrémité aval (114,294) de la chambre est située à un niveau plus élevé que ladite extrémité amont (112, 286) de la chambre, le sens d'écoulement du fluide à travers au moins une partie de ladite chambre étant ainsi sensiblement ascendant et sensiblement opposé au sens d'écoulement du fluide entre la source de fluide et le patient.

2. Boîtier suivant la revendication 1, dans lequel ledit réceptacle (80) comprend également ledit segment de sortie (116).

3. Boîtier (306) suivant la revendication 1, dans lequel ladite cartouche (310) comprend une paroi (314) définissant ladite chambre (312) et une embouchure (320) de ladite chambre, et elle comprend en outre un bouchon perçable et refermable (318) placé de façon étanche dans ladite embouchure, ladite cartouche pouvant s'accoupler au reste dudit boîtier de sorte que ladite chambre se trouve en communication de fluide à la fois avec ladite entrée (94) et ledit segment de sortie (116) à travers ledit bouchon unique.

4. Boîtier suivant la revendication 3, comprenant en outre une partie intermédiaire séparée (308) prévue pour une connexion entre ladite cartouche (310) et ledit réceptacle (80) et assurant la communication de fluide entre ces derniers.

5. Boîtier suivant la revendication 3 ou 4, dans lequel ladite cartouche comprend en outre une bande métallique (322) autour de la périphérie extérieure d'un goulot (316) de ladite paroi (314) définissant ladite embouchure, la bande bloquant ledit bouchon (318) dans ladite cartouche.

6. Boîtier suivant la revendication 3, 4 ou 5, dans lequel ladite cartouche est un flacon de médicament (310).

7. Boîtier suivant l'une quelconque des revendications précédentes, comprenant en outre :
   a) un premier segment de connexion (122 et 122a, 290, 324), disposé entre ladite entrée (94) et ladite extrémité amont (112,286,336) de la chambre et assurant la communication de fluide entre ces dernières ; et
   b) un deuxième segment de connexion (124 et 124a, 298, 326), disposé entre ladite extrémité aval (114,286,338) de la chambre et ladite sortie (96) et assurant la communication de fluide entre ces dernières.

8. Boîtier suivant la revendication 7, dans lequel ledit deuxième segment de connexion (298) comprend ledit segment de sortie (296).

9. Boîtier suivant la revendication 7 ou 8, dans lequel ledit deuxième segment de connexion (124,124a) est disposé entre ladite extrémité aval (114) de la chambre et ladite extrémité amont (118) du segment de sortie et assure la communication de fluide entre celles-ci.

10. Boîtier suivant la revendication 7, dans lequel ledit premier segment de connexion (324) comprend une extrémité de segment (340) et dans lequel ledit deuxième segment de connexion (328) comprend une extrémité de segment (342), lesdites extrémités (340, 342) étant disposées dans ladite chambre (312).

11. Boîtier suivant la revendication 10, dans lequel ladite extrémité de segment (342) du deuxième segment de connexion (326) et ladite extrémité de segment (340) du premier segment de connexion (324) sont disposées à des niveaux différents dans ladite chambre (312), ladite extrémité de segment (342) du deuxième segment de connexion (326) étant en aval de ladite extrémité de segment (340) du premier segment de connexion (324) et étant plus proche de ladite extrémité aval (338) de la chambre que ladite extrémité de segment (340) du premier segment de connexion (324).

12. Boîtier suivant la revendication 7, 8, 9 10 ou 11, comprenant en outre :
   a) des moyens perforables (126,128) disposés dans lesdits premier et deuxième segments de connexion (122 et 122a, 124 et 124a) et obturant ces deux segments; et
   b) des moyens de perforation (130,132) prévus dans lesdits premier et deuxième segments de connexion pour percer lesdits moyens perforables de sorte que, lorsqu'on rapproche ladite cartouche et ledit réceptacle l'un de l'autre, lesdits moyens de perforation percent les dits moyens perforables, ce qui met ladite entrée (94) et ledit segment de sortie (116) en communication de fluide.

13. Boîtier suivant la revendication 12, dans lequel ledit premier segment de connexion (122,122a) comprend un guidage d'écoulement (134) disposé dans ledit réceptacle (80) et comportant :
   a) un tube (136) ayant une extrémité amont (138), raccordée de façon étanche au fluide à ladite extrémité aval (104) du segment de réceptacle (12), et une extrémité opposée (140) ;
   b) un site d'injection perforable refermable (126) définissant les moyens perforables qui obturent ledit premier segment (122 et 122a) et connecté à ladite extrémité opposée (140) dudit tube ; et

EP 0 203 162 B1

c) un orifice de contournement (142) défini dans ledit tube de sorte que, lorsque ladite cartouche n'est pas fixée audit réceptacle, le fluide entrant dans le dit réceptacle passe dans ledit guidage et sort dudit guidage d'écoulement par ledit orifice de contournement, vers ladite sortie.

14. Boîtier suivant la revendication 12 ou 13, dans lequel lesdits moyens perforables comprennent des sites d'injection élastiques de type caoutchouc (126, 128).

15. Boîtier suivant la revendication 14, dans lequel lesdits moyens perforables comprennent des canules pointues (130,132), lesdites canules faisant partie desdits premier et deuxième segments de connexion (122,124).

16. Boîtier suivant la revendication 13, dans lequel lesdits moyens perforables (130) du premier segment de connexion (122) comprennent une canule pointue (130) de sorte que, lorsqu'on fixe ladite cartouche audit réceptacle, ladite canule (130) perce ledit site d'injection (126) du guidage d'écoulement et le fluide pénétrant dans ledit réceptacle passe dans ledit tube (134) du guidage d'écoulement, pratiquement tout le dit fluide dans ledit tube de guidage d'écoulement passant dans ladite canule (130) et en aval vers ladite sortie (96).

17. Boîtier suivant la revendication 16, dans lequel ledit tube (136) du guidage d'écoulement est élastique et dimensionné de manière à venir en contact étanche autour de la périphérie extérieure de la dite canule creuse (130), ce qui empêche le fluide pénétrant dans ledit tube de guidage d'écoulement (136) de sortir par ledit orifice de contournement (142) lorsque ladite cartouche est fixée audit réceptacle.

18. Boîtier suivant la revendication 17, dans lequel ledit guidage d'écoulement (134) rencontre mais n'obture pas ledit segment de sortie (116).

19. Boîtier suivant la revendication 1, dans lequel lesdits moyens de connexion comprennent le dit segment de sortie (296).

20. Boîtier suivant la revendication 1, dans lequel ledit réceptacle comprend en outre un canal (272) qui coopère avec ledit segment de sortie (296) de sorte que le fluide qui entre dans le segment de réception (252) ne s'échappe pas immédiatement dans la sortie (246) mais entre dans ledit premier segment de connexion (290).

21. Boîtier suivant l' une quelconque des revendications précédentes, dans lequel ledit agent de traitement (108) est sous forme de poudre.

22. Boîtier suivant la revendication 1, dans lequel ladite partie amont (148) de la chambre présente une configuration en entonnoir s'élargissant vers l'aval.

23. Boîtier suivant la revendication 1, dans lequel ladite partie aval (150) de la chambre présente une configuration en entonnoir qui se rétrécit vers l'aval.

24. Boîtier suivant la revendication 1, comprenant en outre des moyens d'élimination d'air (86) dans le conduit de fluide, pour élimines l'air entraîné dans le conduit de fluide, lesdits moyens d'élimination d'air étant placés dans une position qui est l'une de (a) dans ladite chambre et (b) en aval de la dite chambre.

25. Boîtier suivant l'une quelconque des revendications précédentes, dans lequel ladite cartouche (82), lorsqu'elle est fixée audit réceptacle (80),ne comporte pas de moyens pour diriger l'air entraîné dans le conduit de fluide hors de ladite cartouche et vers l'ambiance extérieure du système de distribution intraveineuse, sans que l'air entraîné s'écoule d'abord en aval de ladite cartouche.

26. Boîtier suivant l'une quelconque des revendications 1 à 25, comprenant en outre une partie optiquement transparente de la paroi de la chambre, la dite chambre contenant un indicateur visuel flottant (129), autre que l'agent de traitement, qui change de niveau, du fait de la densité changeante du fluide, à la fin du mélange de l'agent de traitement et de la distribution de l'agent on aval de ladite chambre.

19

**27.** Boîtier suivant l'une quelconque des revendications 1 à,25, comprenant en outre une partie optiquement transparente de la paroi de la chambre, et comportant une pluralité d'indicateurs visuels flottants (129), autres que l'agent de traitement, dans ladite chambre, lesdits indicateurs pouvant flotter dans des fluides de plages de densité différentes de sorte que, lorsque le mélange est presque complet, certains desdits indicateurs visuels commencent à s'enfoncer et de sorte que, après achèvement du mélange et distribution de l'agent de traitement hors de ladite chambre, tous les dits indicateurs visuels sont descendus.

**28.** Boîtier suivant l'une quelconque des revendications 1 à 25, comprenant en outre des parties du boîtier, définissant ledit segment de réception et ladite chambre, qui sont optiquement transparentes, chacune contenant une pluralité d'indicateurs visuels flottants (129), autres que l'agent de traitement, lesdits indicateurs visuels flottants placés dans ledit segment de réception pouvant flotter dans des fluides de plages de densité différentes, et lesdits indicateurs visuels placés dans ladite chambre pouvant flotter dans des fluides de plages de densité différentes mais dans les mêmes plages que lesdits indicateurs placés dans ledit segment de réception, de sorte qu'une confirmation visuelle du mélange complet et de la distribution de l'agent en aval de ladite chambre est obtenue lorsqu 'il reste le même nombre d'indicateurs visuels qui flottent dans ladite chambre et dans ledit segment de réception.

**29.** Boîtier suivant l'une quelconque des revendications 1 à 25, comprenant en outre une partie du boîtier, définissant ledit segment de sortie, qui est optiquement transparente, ledit segment de sortie contenant un indicateur visuel flottant (129), autre que l'agent de traitement, qui change de niveau, du fait du changement de densité du fluide, à la fin du mélange de l'agent de traitement et de la distribution de l'agent en aval dudit segment de sortie.

**30.** Boîtier suivant l'une quelconque des revendications 1 à 25, comprenant en outre une partie de la paroi du segment de sortie qui est optiquement transparente, et comportant une pluralité d'indicateurs visuels flottants (129), autres que l'agent de traitement, dans ledit segment de sortie, lesdits indicateurs pouvant flotter dans des fluides de plages de densité différentes de sorte que, lorsque le mélange est proche de son achèvement, certains desdits indicateurs visuels commencent à tomber et de sorte que, après achèvement du mélange et après distribution de l'agent de traitement hors dudit segment de sortie, tous ces indicateurs visuels sont tombés.

**31.** Boîtier suivant l' une quelconque des revendications 1 à 25, comprenant en outre des parties du boîtier, définissant ledit segment de réception et ledit segment de sortie, qui sont optiquement transparentes, chacun dudit segment de sortie et dudit segment de réception contenant une pluralité d'indicateurs visuels flottants (129), autres que l'agent de traitement, lesdits indicateurs visuels flottants placés dans ledit segment de réception pouvant flotter dans des fluides de plages de densité différentes, et lesdits indicateurs visuels placés dans ledit segment de sortie pouvant flotter dans des fluides de plages de densité différentes mais dans les mêmes plages que lesdits indicateurs dudit segment de réception, de sorte qu'une confirmation visuelle du mélange complet et de la distribution de l'agent en aval dudit segment de sortie est obtenue lorsqu'il reste le même nombre d'indicateurs visuels qui flottent dans ledit segment de sortie et dans ledit segment de réception.

**32.** Réceptacle (80) prévu pour l'utilisation dans un boîtier suivant la revendication 1, à insérer dans un système de distribution intraveineuse comprenant une source de fluide (62) et un conduit de fluide (76), pour distribuer un fluide à partir de la source de fluide, par l'intermédiaire du conduit de fluide, au système intraveineux d'un patient, ledit réceptacle comprenant :
    (a) une entrée (94) prévue pour raccordement à une partie amont du conduit de fluide ;
    (b) un segment de réception de fluide (100) ayant une extrémité amont (102) en communication de fluide avec ladite entrée, et une extrémité aval (104) ;
    (c) une sortie (96) prévue pour raccordement à une partie aval du conduit de fluide ;
    (d) un segment de sortie (116) comportant une extrémité amont (118,300) en communication de fluide avec un site d'injection prévu pour communication avec l'extrémité aval (114) d'une cartouche séparée, et une extrémité aval (120) du segment de sortie en communication avec ladite sortie ; et
    (e) un guidage d'écoulement (134), ledit guidage d'écoulement comprenant
        i) un tube (136) qui a une extrémité amont (138), raccordée de façon étanche au fluide à ladite extrémité aval (104) du segment de réception, et une extrémité opposée (140),
        ii) un site d'injection perforable refermable (126) fixé à ladite extrémité opposée (140) pour

20

communication avec l'extrémité amont (118) de la cartouche séparée, et

iii) un orifice de contournement (142) défini dans ledit tube ;

(f) de sorte que, lorsque la cartouche séparée n'est pas accouplée audit réceptacle, le fluide qui entre dans ledit réceptacle à ladite entrée passe successivement dans ledit segment de réception, ledit tube de guidage d'écoulement, ledit orifice de contournement dudit guidage d'écoulement et ladite sortie, ledit guidage d'écoulement rencontrant mais n'obturant pas ledit segment de sortie ; et

(g) ledit tube de guidage d'écoulement est élastique et assure une étanchéité effective autour de la périphérie extérieure d'une canule de cartouche insérée à travers ledit site d'injection du guidage d'écoulement de sorte que, lorsque la cartouche séparée est fixée audit réceptacle, le fluide qui entre dans ledit réceptacle à ladite entrée passe successivement dans le dit segment de réception, ledit tube de guidage d'écoulement, la canule de cartouche, la cartouche, ledit segment de sortie et ladite sortie, ledit réceptacle étant ainsi pratiquement sens écoulement de fluide à travers ledit orifice de contournement du guidage d'écoulement.

33. Méthode de mélange d'un agent de traitement (108) avec un fluide s'écoulant à partir d'une source de fluide (62) dans un conduit de fluide (76), les opérations comprenant :

(a) la préparation d'un réceptacle (80), ayant une entrée de fluide (94) et une sortie de fluide (96) dans le conduit de fluide, prévu pour recevoir une cartouche séparée (82) et permettant, lorsque la cartouche n'est pas fixée eu réceptacle, au fluide venant de la source de fluide d'être distribué eu patient par l'intermédiaire du conduit de fluide comportant le réceptacle, aucun fluide ne sortant du réceptacle entre l'entrée et la sortie ;

(b) la préparation d'une cartouche séparée (82) définissant une chambre (106) qui contient un agent de traitement (108), la chambre ayant une extrémité amont (112) prévue pour une communication de fluide avec l'entrés (94) du réceptacle lorsque la cartouche est fixée au réceptacle, la chambre ayant en outre une extrémité aval (114),

(c) la création d'un segment de sortie (116) disposé à la fois dans la cartouche et le réceptacle lorsque la cartouche est fonctionnellement fixée au réceptacle, le segment de sortie comportant en outre une extrémité amont (118), prévue pour communication avec l'extrémité aval (114) de la chambre, et une extrémité aval (120) de segment de sortie en communication avec la sortie (96) lorsque la cartouche est fonctionnellement fixée eu réceptacle ; et

(d) la fixation fonctionnelle de la cartouche au réceptacle de manière à :

i) diriger pratiquement tout le liquide à travers l 'entrée du réceptacle, la chambre et le segment de sortie, avant qu'il s'écoule par la sortie du réceptacle ; et

ii) disposer l'extrémité aval (114) de la chambre à un niveau plus élevé que l'extrémité amont (112) de la chambre, et ainsi

iii) diriger l'écoulement de fluide à travers au moins une partie de la chambre dans un sens sensiblement ascendant et sensiblement opposé au sens d'écoulement du fluide venant de la source de fluide,

iv) de sorte que, lorsque le fluide circule à travers la cartouche, l'agent de traitement contenu dans la chambre se mélange au fluide et est distribué avec celui-ci.

34. Méthode suivant la revendication 33, comprenant en outre l'enlèvement de la cartouche du réceptacle pour permettre ensuite un écoulement de fluide à travers le réceptacle.

35. Méthode suivant la revendication 34, comprenant en outre l'accouplement d'une autre cartouche au réceptacle.

36. Méthode suivant la revendication 34, comprenant en outre l'opération de fourniture de l'agent de traitement dans la cartouche sous forme de poudre.

**Patentansprüche**

1. Gehäuse (78, 240, 306), das in ein intravenöses Abgabesystem mit einer Fluidversorgung (62) und einer Fluidleitung (76, 76¹, 76¹¹) zur Abgabe von Flüssigkeit aus der Fluidversorgung durch die Fluidleitung zum intravenösen System eines Patienten einsetzbar ist, wobei das Gehäuse zur Aufnahme eines Heilmittels (108, 236, 344) ausgelegt ist, das mit einem durch die Fluidleitung strömenden Fluid zu mischen ist, wobei das Gehäuse folgendes aufweist:

a) einen Behälter (80, 80¹, 242), der in die Fluidleitung (76, 76¹, 76¹¹) einsetzbar ist und einen mit einem aufstromseitigen Teil der Fluidleitung verbindbaren Einlaß (94, 244), einen mit einem abstromseitigen Teil der Fluidleitung verbindbaren Auslaß (96, 96¹, 246) sowie ein Fluidaufnahmesegment (100, 252) mit einem aufstromseitigen Ende (102, 254) in Fluidverbindung mit dem Einlaß (94, 244) und mit einem abstromseitigen Ende (104, 256) aufweist,

b) eine getrennte Patrone (82, 82¹, 248, 310), die mit dem Behälter verbindbar ist und eine Kammer (106, 258, 312) zur Aufnahme des Heilmittels aufweist, wobei die Kammer ein aufstromseitiges Ende (112, 286, 336) aufweist, das bei Verbindung mit dem Behälter mit dem Einlaß (94, 244) in Fluidverbindung steht, wobei die Kammer ferner ein abstromseitiges Ende (114, 294, 338) aufweist;

c) ein Ausflußsegment (116, 296) mit einem aufstromseitigen Ende (118, 330) in Verbindung mit dem abstromseitigen Ende (114, 294) der Kammer, wobei das Ausflußsegment ein mit dem Auslaß (96) in Verbindung stehendes abstromseitiges Ende (120, 302) hat,

d) so daß während des Strömens von Fluid durch das Gehäuse das Heilmittel in der Kammer sich mit der Flüssigkeit mischt und damit an den Patienten abgegeben wird,

e) Verbindungseinrichtungen (122, 122A & 124, 124A; 278 & 296; 324 & 326) sowohl in dem Behälter als auch in der Patrone zum Befestigen der Patrone an dem Behälter;

f) so daß, wenn die Patrone nicht an dem Behälter befestigt ist, dennoch Fluid aus der Fluidversorgung durch die Fluidleitung (76) einschließlich des Behälters an den Patienten abgegeben werden kann, wobei zwischen dem Behältereinlaß und dem Behälterauslaß keine Flüssigkeit aus dem Behälter austritt;

dadurch gekennzeichnet, daß

g) das aufstromseitige Ende (112, 286) der Kammer mit dem abstromseitigen Ende (104, 256) des Fluidaufnahmesegments in Verbindung steht unter Herstellung der Verbindung mit dem Einlaß (94, 244);

h) so daß, wenn die Kammer mit dem Aufnahmesegment und dem Ausflußsegment in Fluidverbindung steht, praktisch das gesamte aus dem Auslaß ausströmende Fluid sowohl durch das Aufnahmesegment als auch durch die Kammer als auch durch das Ausflußsegment strömt und in dem Gehäuse eine Fluidbahn bildet; und

i) so daß ferner, wenn das Gehäuse in ein intravenöses Abgabesystem eingesetzt ist, das abstromseitige Ende (114, 294) der Kammer höher als das aufstromseitige Ende (112, 286) der Kammer angeordnet ist, so daß die Richtung der Fluidströmung durch wenigstens einen Teil der Kammer allgemein aufwärts und allgemein entgegengesetzt zu der Strömungsrichtung des Fluids von der Fluidversorgung zum Patienten verläuft.

2. Gehäuse nach Anspruch 1, wobei der Behälter (80) auch das Ausflußsegment (116) aufweist.

3. Gehäuse (306) nach Anspruch 1, wobei die Patrone (310) eine die Kammer (312) sowie eine Öffnung (320) zu der Kammer definierende Wand (314) und ferner einen durchstechbaren, wieder abdichtbaren Stopfen (318) aufweist, der in der Öffnung angeordnet ist und sie dicht verschließt, wobei die Patrone in das übrige Gehäuse so einsteckbar ist, daß die Kammer durch den einzigen Stopfen sowohl mit dem Einlaß (94) als auch mit dem Ausflußsegment (116) in Fluidverbindung steht.

4. Gehäuse nach Anspruch 3, das ferner einen getrennten Zwischenteil (308) aufweist zur Herstellung einer Verbindung sowie einer Fluidverbindung zwischen der Patrone (310) und dem Behälter (80).

5. Gehäuse nach Anspruch 3 oder 4, wobei die Patrone ferner ein Metallband (322) um den Außenumfang eines Halses (316) der die Öffnung definierenden Wand (314) herum aufweist, wobei das Band den Stopfen (318) in der Patrone festlegt.

6. Gehäuse nach Anspruch 3, 4 oder 5, wobei die Patrone eine Arzneimittelampulle (310) ist.

7. Gehäuse nach einem der vorhergehenden Ansprüche, das ferner folgendes aufweist:

a) ein erstes Anschlußsegment (122 & 122A, 290, 324), das zwischen dem Einlaß (94) und dem aufstromseitigen Ende (112, 286, 336) der Kammer angeordnet ist und eine Fluidverbindung dazwischen herstellt; und

b) ein zweites Anschlußsegment (124 & 124A, 298, 326), das zwischen dem abstromseitigen Ende (114, 286, 338) der Kammer und dem Auslaß (96) angeordnet ist und eine Fluidverbindung dazwischen herstellt.

8. Gehäuse nach Anspruch 7, wobei das zweite Anschlußsegment (298) das Ausflußsegment (296) aufweist.

9. Gehäuse nach Anspruch 7 oder 8, wobei das zweite Anschlußsegment (124, 124A) zwischen dem abstromseitigen Ende (114) der Kammer und dem aufstromseitigen Ende (118) des Ausflußsegments angeordnet ist und eine Fluidverbindung dazwischen herstellt.

10. Gehäuse nach Anspruch 7, wobei das erste Anschlußsegment (324) ein Segmentende (340) und das zweite Anschlußsegment (326) ein Segmentende (342) aufweist, wobei die Enden (340, 342) in der Kammer (312) angeordnet sind.

11. Gehäuse nach Anspruch 10, wobei das Segmentende (342) des zweiten Anschlußsegments (326) und das Segmentende (340) des ersten Anschlußsegments (324) in der Kammer (312) in verschiedenen Höhen angeordnet sind, wobei das Segmentende (342) des zweiten Anschlußsegments (326) an der Abstromseite des Segmentendes (340) des ersten Anschlußsegments (324) und dem abstromseitigen Ende (338) der Kammer näher angeordnet ist als das Segmentende (340) des ersten Anschlußsegments (324).

12. Gehäuse nach Anspruch 7, 8, 9, 10 oder 11, das ferner folgendes aufweist:
a) durchstechbare Einrichtungen (126, 128), die im ersten und im zweiten Anschlußsegment (122 & 122A, 124 & 124A) angeordnet sind und die beiden Anschlußsegmente blockieren; und
b) Durchstecheinrichtungen (130, 132) sowohl im ersten als auch im zweiten Anschlußsegment, die die durchstechbaren Einrichtungen durchstechen, so daß, wenn die Patrone und der Behälter aneinandergedrückt sind, die Durchstecheinrichtungen die durchstechbaren Einrichtungen durchdringen und den Einlaß (94) und das Ausflußsegment (116) in Fluidverbindung bringen.

13. Gehäuse nach Anspruch 12, wobei das erste Anschlußsegment (122, 122A) eine Strömungsleiteinrichtung (134) aufweist, die in dem Behälter (80) angeordnet ist und aufweist:
a) ein Röhrchen (136) mit einem aufstromseitigen Ende (138), das mit dem abstromseitigen Ende (104) des Aufnahmesegments fluiddicht verbunden ist, und mit einem entgegengesetzten Ende (140);
b) eine durchstechbare, wieder abdichtbare, die durchstechbaren Einrichtungen definierende Injektionsstelle (126), die das erste Segment (122 & 122A) blockiert und mit dem entgegengesetzten Ende (140) des Röhrchens verbunden ist; und
c) eine Strömungsbypassöffnung (142), die in dem Röhrchen definiert ist, so daß, wenn die Patrone nicht an dem Behälter befestigt ist, in den Behälter strömendes Fluid in die Strömungsleiteinrichtung und an der Strömungsbypassöffnung aus der Strömungsleiteinrichtung zum Auslaß strömt.

14. Gehäuse nach Anspruch 12 oder 13, wobei die durchstechbaren Einrichtungen elastische, gummiähnliche Injektionsstellen (126, 128) aufweisen.

15. Gehäuse nach Anspruch 14, wobei die durchstechbaren Einrichtungen spitze Kanülen (130, 132) aufweisen, die Teil des ersten und zweiten Anschlußsegments (122, 124) sind.

16. Gehäuse nach Anspruch 13, wobei die durchstechbare Einrichtung (130) in dem ersten Anschlußsegment (122) eine spitze Kanüle (130) aufweist, so daß bei am Behälter befestigter Patrone die Kanüle (130) die Injektionsstelle (126) der Strömungsleiteinrichtung durchsticht und in den Behälter strömendes Fluid in das Strömungsleitröhrchen (136) strömt, wobei praktisch das gesamte Fluid in dem Strömungsleitröhrchen in die Kanüle (130) und stromabwärts zum Auslaß (96) strömt.

17. Gehäuse nach Anspruch 16, wobei das Strömungsleitröhrchen (136) elastisch und von solcher Größe ist, daß es um den Außenumfang der Hohlkanüle (130) herum eine Abdichtung bildet und in das Strömungsleitröhrchen (136) strömendes Fluid am Ausströmen aus der Strömungsbypassöffnung (142) hindert, wenn die Patrone an dem Behälter befestigt ist.

**18.** Gehäuse nach Anspruch 17, wobei die Strömungsleiteinrichtung (134) das Ausflußsegment (116) schneidet, aber nicht verschließt.

**19.** Gehäuse nach Anspruch 1, wobei die Verbindungseinrichtung das Ausflußsegment (296) aufweist.

**20.** Gehäuse nach Anspruch 1, wobei der Behälter ferner einen Kanal (272) aufweist, der so mit dem Ausflußsegment (296) zusammenwirkt, daß in das Aufnahmesegment (252) eintretendes Fluid nicht sofort in den Auslaß (246) ausströmt, sondern in das erste Anschlußsegment (290) eintritt.

**21.** Gehäuse nach einem der vorhergehenden Ansprüche, wobei das Heilmittel (108) pulverförmig ist.

**22.** Gehäuse nach Anspruch 1, wobei der aufstromseitige Teil (148) der Kammer eine trichterförmige Konfiguration aufweist, die in Abstromrichtung weiter wird.

**23.** Gehäuse nach Anspruch 1, wobei der abstromseitige Teil (150) der Kammer eine trichterförmige Konfiguration aufweist, die in Abstromrichtrnng enger wird.

**24.** Gehäuse nach Anspruch 1, das ferner in der Fluidleitung eine Entlüftungseinrichtung (86) zum Entfernen von in die Fluidleitung mitgenommener Luft aufweist, wobei die Entlüftungseinrichtung in einer Lage angeordnet ist, die entweder (a) in der Kammer oder (b) an der Abstromseite der Kammer liegt.

**25.** Gehäuse nach einem der vorhergehenden Ansprüche, wobei die Patrone (82), wenn sie an dem Behälter (80) befestigt ist, keinerlei Mittel zum Leiten von in die Fluidleitung mitgenommener Luft aus der Patrone in die Umgebung außerhalb des intravenösen Abgabesystems hat, ohne daß die mitgenommene Luft zuerst stromabwärts von der Patrone strömt.

**26.** Gehäuse nach einem der Ansprüche 1-25, das ferner einen optisch transparenten Teil der Kammerwand aufweist, wobei die Kammer eine sich von dem Heilmittel unterscheidende, schwimmende Sichtanzeigeeinrichtung (129) enthält, die aufgrund der sich ändernden relativen Dichte des Fluids bei Beendigung des Mischens des Heilmittels und der Abgabe des Mittels an der Abstromseite der Kammer ihre Höhe ändert.

**27.** Gehäuse nach einem der Ansprüche 1-25, das ferner einen optisch transparenten Teil der Kammerwand aufweist und in der Kammer eine Vielzahl von sich von dem Heilmittel unterscheidenden, schwimmenden Anzeigeeinrichtungen (129) enthält, wobei die Anzeigeeinrichtungen in Fluiden mit verschiedenen Bereichen relativer Dichte schwimmen können, so daß bei sich nähernder Beendigung des Mischens gewisse Sichtanzeigeeinrichtungen zu fallen beginnen und nach Beendigung des Mischens und nach Abgabe des Heilmittels aus der Kammer sämtliche Sichtanzeigeeinrichtungen gefallen sind.

**28.** Gehäuse nach einem der Ansprüche 1-25, das ferner optisch transparente Teile des Gehäuses aufweist, die das Aufnahmesegment und die Kammer definieren, wobei jeder Teil eine Vielzahl von sich von dem Heilmittel unterscheidenden, schwimmenden Sichtanzeigeeinrichtungen (129) enthält und die schwimmenden Sichtanzeigeeinrichtungen in dem Aufnahmesegment in Fluiden mit verschiedenen Bereichen relativer Dichte schwimmen können und die Sichtanzeigeeinrichtungen in der Kammer in Fluiden mit verschiedenen Bereichen relativer Dichte schwimmen können, die jedoch gleich den Bereichen der Anzeigeeinrichtungen in dem Aufnahmesegment sind, wobei eine Sichtbestätigung des vollständigen Mischens und der Abgabe des Mittels an der Abstromseite der Kammer erfolgt, wenn in der Kammer die gleiche Anzahl von Sichtanzeigeeinrichtungen weiter schwimmt wie in dem Aufnahmesegment.

**29.** Gehäuse nach einem der Ansprüche 1-25, das ferner einen optisch transparenten Teil des Gehäuses aufweist, der das Ausflußsegment definiert, wobei das Ausflußsegment eine sich von dem Heilmittel unterscheidende, schwimmende Sichtanzeigeeinrichtung (129) enthält, die aufgrund der sich ändernden relativen Dichte in dem Fluid bei Beendigung des Mischens des Heilmittels und der Abgabe des Mittels an der Abstromseite des Ausflußsegments ihre Höhe ändert.

**30.** Gehäuse nach einem der Ansprüche 1-25, das ferner einen optisch transparenten Teil der Ausflußsegmentwand aufweist und in dem Ausflußsegment eine Vielzahl von sich von dem Heilmittel unterscheidenden, schwimmenden Sichtanzeigeeinrichtungen (129) enthält, wobei die Anzeigeeinrichtungen in Fluiden mit verschiedenen Bereichen relativer Dichte schwimmen können, so daß bei sich nähernder Beendigung des Mischens gewisse Sichtanzeigeeinrichtungen zu fallen beginnen und nach Beendigung des Mischens und nach Abgabe des Heilmittels aus dem Ausflußsegment sämtliche Sichtanzeigeeinrichtungen gefallen sind.

**31.** Gehäuse nach einem der Ansprüche 1-25, das ferner optisch transparente Teile des Gehäuses aufweist, die das Aufnahmesegment und das Ausflußsegment definieren, wobei sowohl das Ausflußsegment als auch das Aufnahmesegment eine Vielzahl von sich von dem Heilmittel unterscheidenden, schwimmenden Sichtanzeigeeinrichtungen (129) enthalten, wobei die schwimmenden Sichtanzeigeeinrichtungen in dem Aufnahmesegment in Fluiden mit verschiedenen Bereichen relativer Dichte schwimmen können und die Sichtanzeigeeinrichtungen in dem Ausflußsegment in Fluiden mit verschiedenen Bereichen relativer Dichte schwimmen können, die jedoch gleich den Bereichen der Anzeigeeinrichtungen in dem Aufnahmesegment sind, wobei eine Sichtbestätigung des vollständigen Mischens und der Abgabe des Mittels an der Abstromseite des Ausflußsegments erfolgt, wenn in dem Ausflußsegment die gleiche Anzahl von Sichtanzeigeeinrichtungen weiter schwimmt wie in dem Aufnahmesegment.

**32.** Behälter (80) zur Verwendung in einem Gehäuse nach Anspruch 1 zum Einsetzen in ein intravenöses Abgabesystem mit einer Fluidversorgung (62) und einer Fluidleitung (76) zur Abgabe von Fluid aus der Fluidversorgung durch die Fluidleitung zum intravenösen System eines Patienten, wobei der Behälter folgendes aufweist:

a) einen mit einem aufstromseitigen Teil der Fluidleitung verbindbaren Einlaß (94);

b) ein Fluidaufnahmesegment (100) mit einem aufstromseitigen Ende (102) in Fluidverbindung mit dem Einlaß und mit einem abstromseitigen Ende (104);

c) einen mit einem abstromseitigen Teil der Fluidleitung verbindbaren Auslaß (96);

d) ein Ausflußsegment (116) mit einem aufstromseitigen Ende (118, 300) in Fluidverbindung mit einer Injektionsstelle, die mit dem abstromseitigen Ende (114) einer getrennten Patrone verbindbar ist, und mit einem abstromseitigen Ende (120) in Verbindung mit dem Auslaß; und

e) eine Strömungsleiteinrichtung (134), die aufweist:

i) ein Röhrchen (136) mit einem aufstromseitigen Ende (138), das mit dem abstromseitigen Ende (104) des Aufnahmesegments fluiddicht verbunden ist, und mit einem entgegengesetzten Ende (140),

ii) eine durchstechbare, wieder abdichtbare Injektionsstelle (126), die zur Verbindung mit dem aufstromseitigen Ende (118) der getrennten Patrone an dem entgegengesetzten Ende (140) befestigt ist, und

iii) eine in dem Röhrchen definierte Strömungsbypassöffnung (142);

f) so daß, wenn die getrennte Patrone nicht mit dem Behälter verbunden ist, an dem Einlaß in den Behälter eintretendes Fluid aufeinanderfolgend durch das Aufnahmesegment, das Strömungsleitrährchen, die Bypassöffnung der Strömungsleiteinrichtung und den Auslaß strömt, wobei die Strömungsleiteinrichtung das Ausflußsegment schneidet, aber nicht verschließt,

g) wobei das Strömungsleitrährchen elastisch ist und um den Außenumfang einer durch die Injektionsstelle der Strömungsleiteinrichtung eingeführten Patronenkanüle eine wirksame Abdichtung bildet, so daß, wenn die getrennte Patrone an dem Behälter befestigt ist, an dem Einlaß in den Behälter eintretendes Fluid aufeinanderfolgend durch das Aufnahmesegment, das Strömungsleitrährchen, die Patronenkanüle, die Patrone, das Ausflußsegment und den Auslaß strömt, wodurch in dem Behälter praktisch keine Fluidströmung durch die Bypassöffnung der Strömungsleiteinrichtung geht.

**33.** Verfahren zum Mischen eines Heilmittels (108) mit einem aus einer Fluidversorgung (62) durch eine Fluidleitung (76) strömenden Fluid, das folgende Schritte aufweist:

a) Vorsehen eines Behälters (80), der einen Fluideinlaß (94) und einen Fluidauslaß (96) in der Fluidleitung aufweist, an dem eine getrennte Patrone (82) befestigbar ist und der, wenn die Patrone nicht an dem Behälter befestigt ist, die Abgabe von Fluid aus der Fluidversorgung durch die Fluidleitung einschließlich des Behälters an den Patienten zuläßt, wobei zwischen dem Einlaß und dem Auslaß kein Fluid aus dem Behälter austritt;

b) Vorsehen eine getrennten Patrone (82), die eine ein Heilmittel (108) enthaltende Kammer (106)

25

definiert, wobei die Kammer ein aufstromseitiges Ende (112) aufweist, das mit dem Behältereinlaß (94) in Fluidverbindung bringbar ist, wenn die Patrone an dem Behälter befestigt ist, und wobei die Kammer ferner ein abstromseitiges Ende (114) aufweist;

c) Vorsehen eines Ausflußsegments (116), das bei betriebsmäßig an dem Behälter befestigter Patrone sowohl in der Patrone als auch in dem Behälter angeordnet ist, wobei das Ausflußsegment ferner ein aufstromseitiges Ende (118), das mit dem abstromseitigen Ende (114) der Kammer verbindbar ist, und ein abstromseitiges Ende (120) aufweist, das mit dem Auslaß (96) in Verbindung steht, wenn die Patrone betriebsmäßig an dem Behälter befestigt ist; und

d) betriebsmäßiges Befestigen der Patrone an dem Behälter wodurch:

i) praktisch die gesamte Flüssigkeit durch den Behältereinlaß, die Kammer und das Ausflußsegment geleitet wird, bevor sie aus dem Behälterauslaß ausströmt; und

ii) das abstromseitige Ende (114) der Kammer höher als das aufstromseitige Ende (112) der Kammer angeordnet wird, wodurch:

iii) eine Fluidströmung durch wenigstens einen Teil der Kammer allgemein aufwärts und allgemein entgegengesetzt zu der Strömungsrichtung des Fluids von der Fluidversorgung gerichtet wird;

iv) so daß während des Strömens von Fluid durch die Patrone das Heilmittel in der Kammer sich mit dem Fluid mischt und damit abgegeben wird.

34. Verfahren nach Anspruch 33, das ferner folgenden Schritt aufweist: Abnehmen der Patrone von dem Behälter, um anschließend eine Fluidströmung durch den Behälter zuzulassen.

35. Verfahren nach Anspruch 34, das ferner folgenden Schritt aufweist: Verbinden einer anderen Patrone mit dem Behälter.

36. Verfahren nach Anspruch 34, das ferner folgenden Schritt aufweist: Bereitstellen des Heilmittels in der Patrone in Pulverform.

**FIG. 1**

**FIG. 2**
PRIOR ART

FIG. 3

FIG. 4

FIG. 5

FIG. 6

# FIG. 7

FIG. 8